# EUROPÄISCHE PATENTSCHRIFT

(11) **EP 0 530 647 B1**
(45) Veröffentlichungstag und Bekanntmachung des Hinweises auf die Patenterteilung: **28.01.1998**
(21) Anmeldenummer: 92114464.8
(22) Anmeldetag: 25.08.1992
(51) Int. Cl.: C08F 4/602, C08F 10/00

(54) **Metallocene, diese enthaltende Katalysatoren und Verfahren zur Herstellung eines Olefinpolymers unter Verwendung dieser Katalysatoren**
Metallocenes, catalysts containing these and process for the manufacture of olefinic polymers by using these catalysts
Metallocènes, catalyseurs contenant ces metallocénes et procédé de préparation de polymères oléfiniques en utilisant ces catalyseurs

(30) Priorität: 26.08.1991 DE 4128238
(43) Veröffentlichungstag der Anmeldung: 10.03.1993
(60) Teilanmeldung: 97101674.6
(73) Patentinhaber: HOECHST AKTIENGESELLSCHAFT, 65926 Frankfurt am Main (DE)
(72) Erfinder: Spaleck, Walter, Dr., W-6237 Liederbach (DE); Rohrmann, Jürgen, Dr., W-6233 Kelkheim (Taunus) (DE); Antberg, Martin, Dr., W-6238 Hofheim am Taunus (DE)

(56) Entgegenhaltungen:
- EP-A- 0 344 887
- EP-A- 0 399 348
- EP-A- 0 433 989
- EP-A- 0 433 990
- EP-A- 0 442 725
- EP-A- 0 485 822
- EP-A- 0 485 823
- EP-A- 0 529 908

## Beschreibung

Die vorliegende Erfindung bezieht sich auf ein Verfahren zur Herstellung von Olefinpolymeren mit enger Molmassenverteilung, variabler Molmasse und, im Falle prochiraler Monomere, variabler Mikrostruktur der Kette, sowie die in dem Verfaben eingesetsten Metallocene.

Polyolefine mit hoher Molmasse besitzen insbesondere Bedeutung für die Herstellung von Folien, Platten oder Hohlkörpern wie z.B. Rohre oder Formteile.

Polyolefine mit niedriger Molmasse besitzen Bedeutung für die Herstellung von Additiven oder Gleitmitteln.

Aus der Literatur sind lösliche Metallocenverbindungen auf Basis von Bis(cyclopentadienyl)zirkon-alkyl bzw. halogenid in Kombination mit oligomeren Aluminoxanen bekannt. Mit diesen Systemen können Ethylen mit guter und Propylen mit mäßiger Aktivität polymerisiert werden. Man erhält Polyethylen enger Molmassenverteilung und mittlerer Molmasse, das erhaltene Polypropylen ist ataktisch und hat eine sehr niedrige Molmasse.

Die Herstellung von isotaktischem Polypropylen gelingt mit Hilfe des Ethylen-bis(4,5,6,7-tetrahydro-1-indenyl)zirkondichlorids zusammen mit einem Aluminoxan in einer Suspensionspolymerisation (vgl. EP-A 185 918). Das Polymer besitzt eine enge Molmassenverteilung. Besonderer Nachteil dieses Verfahrens ist jedoch, daß bei technisch interessanten Polymerisationstemperaturen nur Polymere mit sehr niedriger Molmasse hergestellt werden können.

Es wurde auch eine spezielle Voraktivierungsmethode des Metallocens mit einem Aluminoxan vorgeschlagen, welche zu einer beachtlichen Steigerung der Aktivität des Katalysatorsystems und zu einer deutlichen Verbesserung der Kornmorphologie des Polymeren führt (vgl. DE-OS 37 26 067).

Weiterhin sind Katalysatoren auf Basis Ethylenbisindenylhafniumdichlorid und Ethylen-bis(4,5,6,7-tetrahydro-1-indenyl)hafniumdichlorid und Methylaluminoxan bekannt, mit denen durch Suspensionspolymerisation höhermolekulare Polypropylene hergestellt werden können (vgl. J. Am. Chem. Soc. 109 (1987) 6544). Unter technisch relevanten Polymerisationsbedingungen ist jedoch die Kornmorphologie der derart erzeugten Polymeren nicht befriedigend und die Aktivität der eingesetzten Katalysatoren vergleichsweise gering. Verbunden mit den hohen Katalysatorkosten ist somit mit diesen Systemen eine kostengünstige Polymerisation nicht möglich.

Die letztgenannten Probleme werden im Grundsatz gelöst durch die Verwendung verbrückter Metallocen-Katalysatorsysteme, die in 2-Stellung zur Brücke an beiden aromatischen Liganden eine Alkyl- oder Arylgruppe tragen. Solche Systeme sind in der Z A 91/8925 beschrieben.

Die letztgenannten Katalysatoren weisen in ihren Eigenschaften oder Eigenschaftskombinationen aber noch gewisse Defizite auf, wenn man eine besonders breite Anwendbarkeit für verschiedene Polymerisationsaufgaben und eine technisch und ökonomisch günstige Vorgehensweise in Betracht zieht.

In EP 442 725 und EP 529 908 werden Metallocen-Katalysatoren beschrieben, die als Liganden substituierte Indenylgruppen aufweisen.

Insbesondere ist es erwünscht,
- bei hoher Polymerisationstemperatur, beispielsweise 70°C, zu polymerisieren, weil dann die Katalysatoraktivität hoch ist und zur Abführung der Polymerisationswärme weniger Kühlmedium erforderlich ist als bei niedriger Polymerisationstemperatur,
- bei dieser hohen Polymerisationstemperatur Polyolefine verschieden hoher Molmassen erzeugen zu können, ohne daß Wasserstoff als Molmassenregler benutzt werden muß (die so erzeugten Polymere weisen ungesättigte Endgruppen auf, die für chemische Funktionalisierungen benutzt werden können)
- bei dieser hohen Polymerisationstemperatur in der stereospezifischen Polymerisation unterschiedliche stereotaktische Sequenzlängen erzeugen zu können, die sich beispielsweise bei isotaktischem Polypropylen in unterschiedlichen Schmelzpunkten und weiteren Eigenschaftsunterschieden auswirken
- eine Morphologie des Polymerpulvers mit durchschnittlichen Korngrößen > 1000 µm zu erhalten, da solche Pulver ohne Granulierung direkt auf Verarbeitungsmaschinen beaufschlagt werden können.

Es wurde nun gefunden, daß diese Aufgaben unter Verwendung von in der Ligandsphäre in bestimmter Weise substituierten, verbrückten Metallocenen gelöst werden können.

Die Erfindung betrifft somit ein Verfahren zur Herstellung eines Olefinpolymers durch Polymerisation oder Copolymerisation eines Olefins der Formel R^{a}-CH =CH-R^{b}, worin R^{a} und R^{b} gleich oder verschieden sind und ein Wasserstoffatom oder einen Kohlenwasserstoffrest mit 1 bis 14 C-Atomen bedeuten, oder R^{a} und R^{b} bilden mit den sie verbindenden Atomen einen Ring, bei einer Temperatur von -60 bis 200°C, bei einem Druck von 0,5 bis 100 bar, in Lösung, in Suspension oder in der Gasphase, in Gegenwart eines Katalysators, welcher aus einem Metallocen als Übergangsmetallverbindung und einem Cokatalysator gebildet wird, dadurch gekennzeichnet, daß das Metallocen eine Verbindung der Formel I ist, 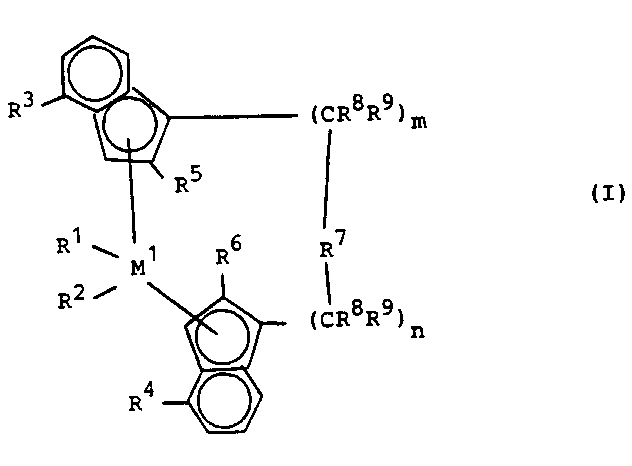 worin
- M¹: ein Metall der Gruppe IVb, Vb oder Vlb des Periodensystems ist,
- R¹ und R²: gleich oder verschieden sind und Wasserstoff, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
- R³ und R⁴: gleich oder verschieden sind und ein Halogenatom, eine C₁-C₁₀-Alkylgruppe, die halogeniert sein kann, eine C₆-C₁₀-Arylgruppe, einen -NR₂¹⁰, -SR¹⁰, -OSiR₃¹⁰, SiR₃¹⁰ oder -PR₂¹⁰-Rest bedeuten, worin R¹⁰ ein Halogenatom, eine C₁-C₁₀-Alkylgruppe, oder eine C₆-C₁₀-Arylgruppe, ist,
- R⁵ und R⁶: gleich oder verschieden sind und die für R³ und R⁴ genannte Bedeutung haben,
- R⁷: 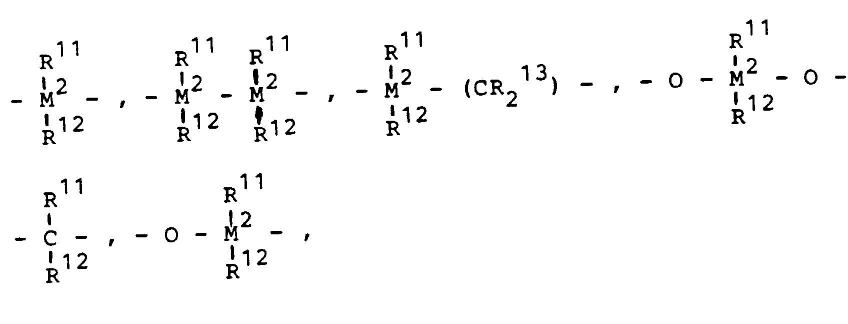 =BR¹¹, =AlR¹¹, -Ge-, -Sn-, -O-, -S-, =SO, =SO₂, =NR¹¹, =CO, =PR¹¹ oder =P(O)R¹¹ ist,
wobei
- R¹¹, R¹² und R¹³: gleich oder verschieden sind und ein Wasserstoffatom, ein Halogenatom, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden, oder R¹¹ oder R¹² mit R⁸ oder R⁹ jeweils zusammen mit den sie verbindenden Atomen einen Ring bilden,
- M²: Silizium, Germanium oder Zinn ist,
- R⁸ und R⁹: gleich oder verschieden sind und die für R¹¹ genannte Bedeutung haben und
- m und n: gleich oder verschieden sind und null, 1 oder 2 sind, wobei m plus n null, 1 oder 2 ist, und das Metallocen der Formel I verschieden von Dimethylsilylenbis(2,4-dimethylindenyl)zirkoniumdichlorid und Ethylenbis(2-methyl-4-methylindenyl)hafniumdichlorid ist.
Alkyl steht für geradkettiges oder verzweigtes Alkyl. Halogen (halogeniert) bedeutet Fluor, Chlor, Brom oder Jod, bevorzugt Fluor oder Chlor.

Gegenstand der vorliegenden Erfindung sind ferner die in dem beschriebenen Verfahren eingesetsten Metallocene.

Der für das erfindungsgemäße Verfahren zu verwendende Katalysator besteht aus einem Cokatalysator und einem Metallocen der Formel I

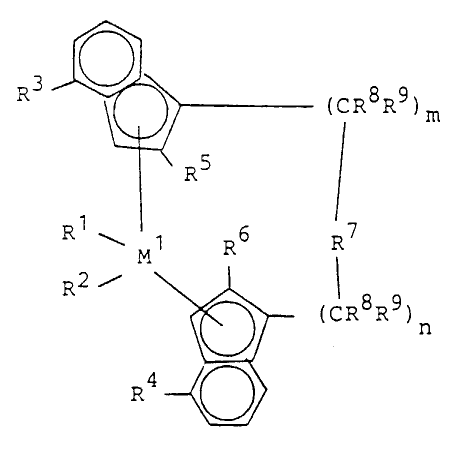

In Formel I ist M¹ ein Metall der Gruppe IVb, Vb oder Vlb des Periodensystems, beispielsweise Titan, Zirkon, Hafnium, Vanadium, Niob, Tantal, Chrom, Molybdän, Wolfram, vorzugsweise Zirkon, Hafnium und Titan.

R¹ und R² sind gleich oder verschieden, bevorzugt gleich, und bedeuten ein Wasserstoffatom, eine C₁-C₁₀-, vorzugsweise C₁-C₃-Alkylgruppe, eine C₁-C₁₀-, vorzugsweise C₁-C₃-Alkoxygruppe, eine C₆-C₁₀-, vorzugsweise C₆-C₈-Arylgruppe, eine C₆-C₁₀- vorzugsweise C₆-C₈-Aryloxygruppe,, eine C₂-C₁₀-, vorzugsweise C₂-C₄-Alkenylgruppe, eine C₇-C₄₀-, vorzugsweise C₇-C₁₀-Arylalkylgruppe, eine C₇-C₄₀-, vorzugsweise C₇-C₁₂-Alkylarylgruppe, eine C₈-C₄₀-, vorzugsweise C₈-C₁₂-Arylalkenylgruppe oder ein Halogenatom, vorzugsweise Chlor.

R³ und R⁴ sind gleich oder verschieden, bevorzugt gleich, und bedeuten ein Halogenatom, bevorzugt ein Chlor-, Brom oder Jodatom, eine C₁-C₁₀-, vorzugsweise C₁-C₆-Alkylgruppe, die halogeniert sein kann, eine C₆-C₁₀-, vorzugsweise C₆-C₈-Arylgruppe, einen -NR₂¹⁰, -SR¹⁰, -OSiR₃¹⁰, -SiR₃¹⁰ oder -PR₂¹⁰-Rest, worin R¹⁰ ein Halogenatom, vorzugsweise Chloratom, oder eine C₁-C₁₀-, vorzugsweise C₁-C₃-Alkylgruppe oder eine C₆-C₁₀-, vorzugsweise C₆-C₈-Arylgruppe ist,

R⁵ und R⁶ sind gleich oder verschieden, bevorzugt gleich, und haben die für R³ und R⁴ beschriebene Bedeutung, mit der Maßgabe, daß R⁵ und R⁶ auch Wasserstoff sein dürfen. Bevorzugt sind R⁵ und R⁶ (C₁-C₄)-Alkyl, das halogeniert sein kann, wie Methyl, Ethyl, Propyl, Isopropyl, Butyl, Isobutyl oder Trifluormethyl, insbesondere Methyl und Ethyl.
- R⁷: ist 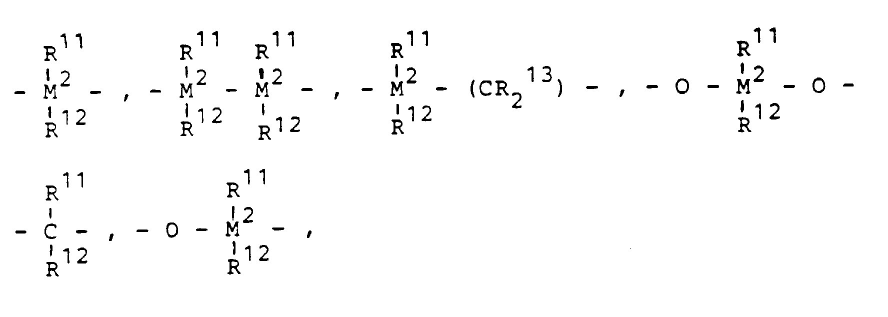 =BR¹¹, =AlR¹¹, -Ge-, -Sn-, -O-, -S-, =SO, =SO₂, =NR¹¹, =CO, =PR¹¹ oder =P(O)R¹¹, wobei R¹¹, R¹² und R¹³ gleich oder verschieden sind und ein Wasserstoffatom, ein Halogenatom, eine C₁-C₁₀-, vorzugsweise C₁-C₄-Alkylgruppe, insbesondere Methylgruppe, eine C₁-C₁₀-Fluoralkylgruppe, vorzugsweise CF₃-Gruppe, eine C₆-C₁₀-, vorzugsweise C₆-C₈-Arylgruppe, eine C₆-C₁₀-Fluorarylgruppe, vorzugsweise Pentafluorphenylgruppe, eine C₁-C₁₀-, vorzugsweise C₁-C₄-Alkoxygruppe, insbesondere Methoxygruppe, eine C₂-C₁₀-, vorzugsweise C₂-C₄-Alkenylgruppe, eine C₇-C₄₀-, vorzugsweise C₇-C₁₀-Arylalkylgruppe, eine C₈-C₄₀-, vorzugsweise C₈-C₁₂-Arylalkenylgruppe oder e ine C₇-C₄₀-, vorzugsweise C₇-C₁₂-Alkylarylgruppe bedeuten, oder R¹¹ und R¹² oder R¹¹ und R¹³ bilden jeweils zusammen mit den sie verbindenden Atomen einen Ring, oder R¹¹ oder R¹² mit R⁸ oder R⁹ jeweils zusammen mit den sie verbindenden Atomen einen Ring bilden,
- M²: ist Silizium, Germanium oder Zinn, bevorzugt Silizium und Germanium.
- R⁷: ist vorzugsweise =CR¹¹R¹², =SiR¹¹R¹², =GeR¹¹R¹², -O-, -S-, =SO, =PR¹¹ oder =P(O)R¹¹.
- R⁸ und R⁹: sind gleich oder verschieden und haben die für R¹¹ genannte Bedeutung.
- m und n: sind gleich oder verschieden und bedeuten null, 1 oder 2, bevorzugt null oder 1, wobei m plus n null, 1 oder 2, bevorzugt null oder 1 ist.

Somit sind die besonders bevorzugten Metallocene die Verbindungen derFormeln A, B und C 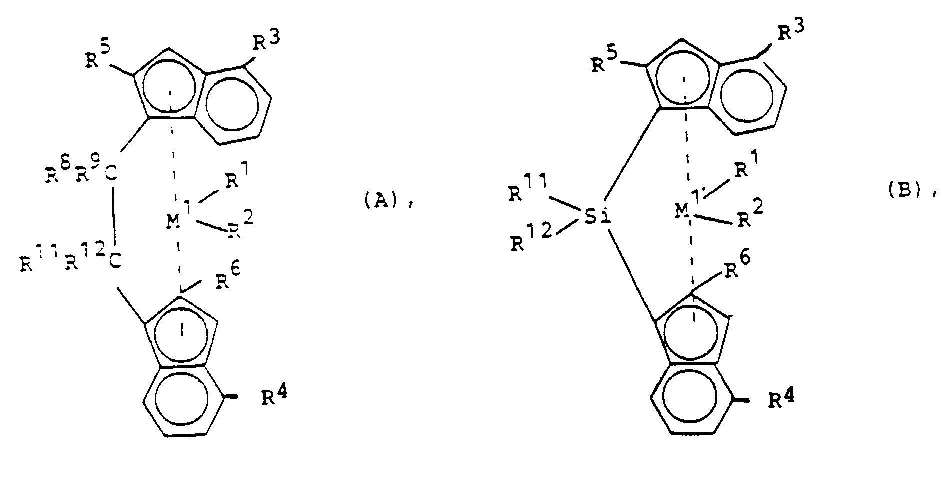 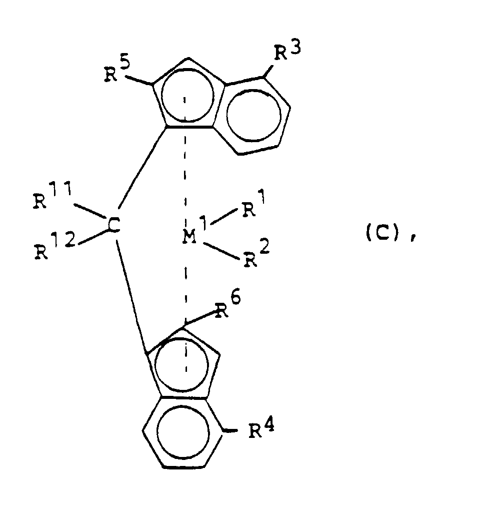 mit
M¹ = Zr, R¹, R²= Methyl, Chlor; R³, R⁴= Methyl, Ethyl, n-Propyl, i-Propyl, n-Butyl,i-Butyl, tert.-Butyl, Neopentyl; R⁵, R⁶= Methyl, Ethyl und R⁸, R⁹, R¹¹, R¹² mit den obengenannten Bedeutungen, insbesondere die in den Beispielen genannten Verbindungen I.
Zur Herstellung isotaktischer Poly-1-olefine werden die chiralen Metallocene als Racemat eingesetzt.
Verwendet werden kann aber auch die reine R- oder S-Form. Mit diesen reinen stereoisomeren Formen ist optisch aktives Polymeres herstellbar. Abgetrennt werden sollte jedoch die meso-Form der Metallocene, da das polymerisationsaktive Zentrum (das Metallatom) in diesen Verbindungen wegen Spiegelsymmetrie am Zentralmetall nicht mehr chiral ist und daher kein hochisotaktisches Polymeres erzeugen kann. Wird die meso-Form nicht abgetrennt, entsteht neben isotaktischen Polymeren auch ataktisches Polymer. Für bestimmte Anwendungen - weiche Formkörper beispielsweise - kann dies durchaus wünschenswert sein.

Die Trennung der Stereoisomeren ist im Prinzip bekannt.

Die vorstehend beschriebenen Metallocene können nach folgendem Reaktionsschema hergestellt werden: 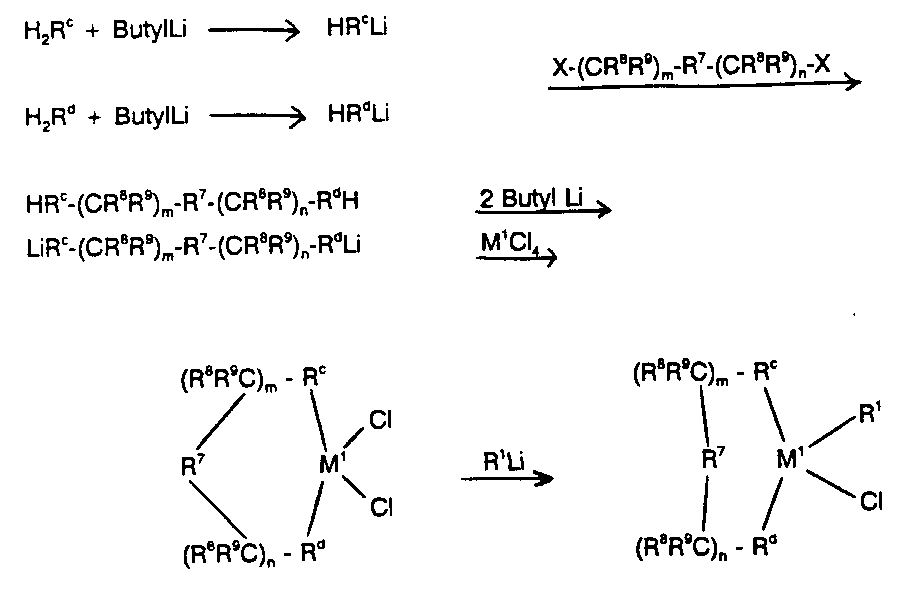 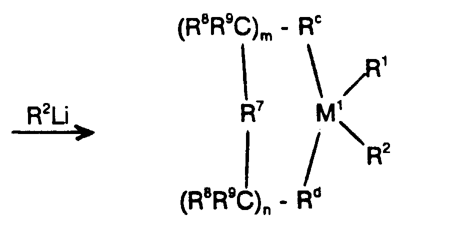 X = Cl, Br, l, O-Tosyl;

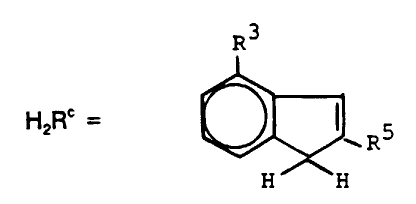

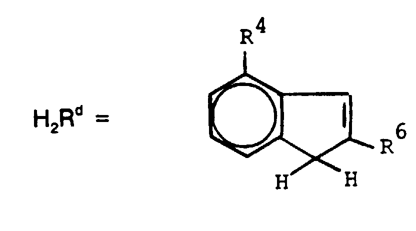

Die Herstellungsverfahren sind literaturbekannt; vgl. Journal of Organometallic Chem. 288 (1985) 63-67, EP-A 320 762 und die Ausführungsbeispiele.

Die Herstellung der als Ausgangssubstanzen dienenden 2,4-substituierten Indene H₂R^{c} bzw. H₂R^{d} ist auf 2 verschiedenen Wegen möglich.
a) Als Ausgangsverbindung dient ein Ketoaldehyd der.im nachstehenden Reaktionsschema angegebenen Formel, dessen Herstellung bekannt ist (Synthesis 1985, 1058).
Die Umsetzung dieses Ketoaldehyds mit Cyclopentadien wird in einem inerten Lösemittel in Gegenwart einer Base durchgeführt. Bevorzugt werden Alkohole wie Methanol, Ethanol oder t-Butanol, insbesondere Methanol, verwendet.

Als Basen lassen sich eine Vielzahl von Verbindungen verwenden. Als Beispiele seien Alkali- und Erdalkalihydroxide, Alkali- und Erdalkalialkoholate, wie Natriummethanolat, Natriumethanolat, Kaliumtertiärbutanolat, Amide wie Lithiumdiisopropylamid oder Amine genannt. Bevorzugt werden Natriumethanolat, Kaliumtertiärbutanolat und Kaliumhydroxid verwendet.

Die Molverhältnisse der Ausgangsverbindungen einschließlich der verwendeten Base können innerhalb weiter Grenzen schwanken. Bevorzugt ist das Molverhältnis Ketoaldehyd:Cyclopentadien:Base = 1:1-1,5:2-3; insbesondere 1:1,1:2,5.

Die Reaktionstemperatur beträgt bevorzugt -40°C bis 100°C, insbesondere 0°C-25°C.

Die Reaktionszeiten schwanken in der Regel zwischen 10 min und 100 h, vorzugsweise zwischen 1 h und 30 h.

Der Substituent in 2-Stellung kann nach Umwandeln des in 4-Position monosubstituierten Indens in das in 4-Position monosubstituierte 2-Indanon nach einer allgemeinen Arbeitsvorschrift (Organic Synthesis, Coll. Vol. V, 1973, 647) durch eine Grignard-Reaktion eingeführt werden. Die anschließende Wasserabspaltung führt zu den 2,4-substituierten Indenen.

Die 2,4-substituierten Indene fallen als Doppelbindungsisomere an, die direkt für die Synthese der entsprechenden Metallocenkomplexe eingesetzt werden können. 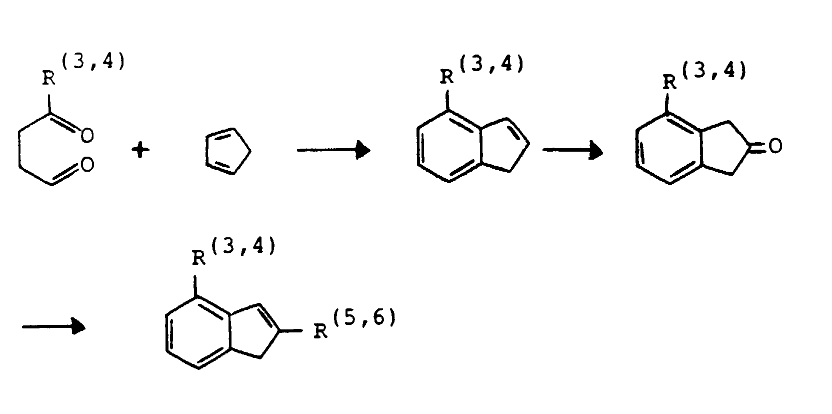 b) Eine andere mögliche und vorteilhafte Strategie verfährt nach folgendem Schema:

Ein in 2-Position substituiertes Benzylhalogenid wird analog eines literaturbekannten Verfahrens (J. Org. chem. 1958, 23, 1437) durch Umsetzen mit einem entsprechend substituierten Malonsäurediester in den disubstituierten Malonsäurediester umgewandelt.

Verseifung des Diesters und Decarboxylierung nach den üblichen Verfahren führt zu einem disubstituierten Propionsäurederivat.

Der Ringschluß zum 2,4-disubstituierten 1-Indanon wird nach Überführen der Carbonsäure in das Carbonsäurechlorid nach gängigen Verfahren durchgeführt.

Reduktion des Ketons nach bekannten Methoden und anschließende Wasserabspaltung liefert die 2,4-disubstituierten Indene.

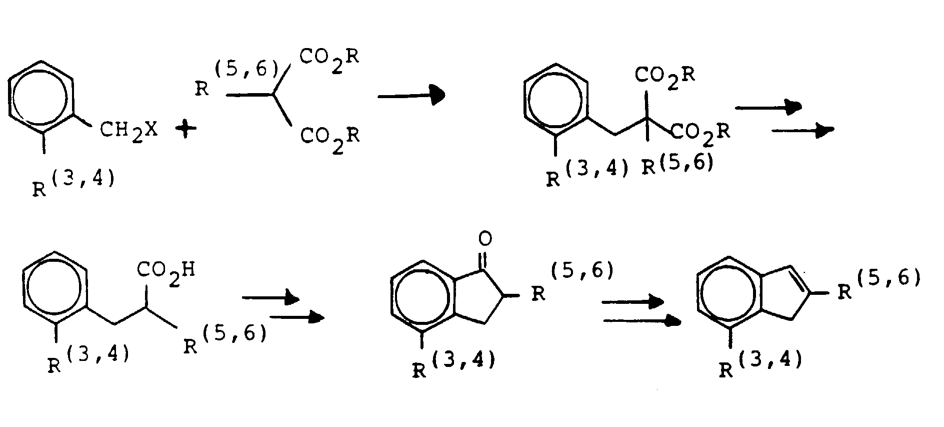

Erfindungsgemäß wird als Cokatalysator bevorzugt ein Aluminoxan der Formel (II) 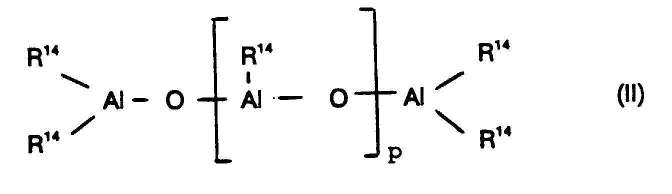 für den linearen Typ und/oder der Formel (III) 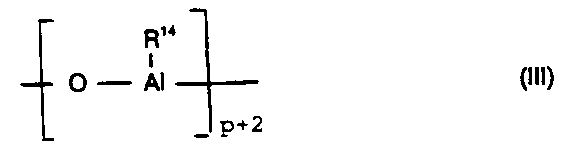 für den cyclischenTyp verwendet, wobei in den Formel (II) und (III) die Reste R¹⁴ gleich oder verschieden sein können und eine C₁-C₆-Alkylgruppe, eine C₆-C₁₈-Arylgruppe oder Wasserstoff bedeuten, und p eine ganze Zahl von 2 bis 50, bevorzugt 10 bis 35 bedeutet.

Bevorzugt sind die Reste R¹⁴ gleich und bedeuten Methyl, Isobutyl, Phenyl oder Benzyl, besonders bevorzugt Methyl.

Sind die Reste R¹⁴ unterschiedlich, so sind sie bevorzugt Methyl und Wasserstoff oder alternativ Methyl und Isobutyl, wobei Wasserstoff bzw. Isobutyl bevorzugt zu 0,01-40 % (Zahl der Reste R¹⁴) enthalten sind.

Das Aluminoxan kann auf verschiedene Arten nach bekannten Verfahren hergestellt werden. Eine der Methoden ist beispielsweise daß eine Aluminiumkohlenwasserstoffverbindung und/oder eine Hydridoaluminiumkohlenwasserstoffverbindung mit Wasser (gasförmig, fest, flüssig oder gebunden - beispielsweise als Kristallwasser) in einem inerten Lösungsmittel (wie z.B. Toluol) umgesetzt wird. Zur Herstellung eines Aluminoxans mit verschiedenen Alkylgruppen R¹⁴ werden entsprechend der gewünschten Zusammensetzung zwei verschiedene Aluminiumtrialkyle (AlR₃ + AlR'₃) mit Wasser umgesetzt (vgl. S. Pasynkiewicz, Polyhedron 9 (1990) 429 und EP-A 302 424).

Die genaue Struktur der Aluminoxane II und III ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, das Metallocen vor dem Einsatz in der Polymerisationsreaktion mit einem Aluminoxan der Formel (II) und/oder (III) vorzuaktivieren. Dadurch wird die Polymerisationsaktivität deutlich erhöht und die Kornmorphologie verbessert.

Die Voraktivierung der Übergangsmetallverbindung wird in Lösung vorgenommen. Bevorzugt wird dabei das Metallocen in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.-%, jeweils bezogen auf die Gesamtlösung. Das Metallocen kann in der gleichen Konzentration eingesetzt werden, vorzugsweise wird es jedoch in einer Menge von 10⁻⁴ - 1 mol pro mol Aluminoxan eingesetzt. Die Voraktivierungszeit beträgt 5 Minuten bis 60 Stunden, vorzugsweise 5 bis 60 Minuten. Man arbeitet bei einer Temperatur von -78°C bis 100°C, vorzugsweise 0 bis 70°C.

Das Metallocen kann auch vorpolymerisiert oder auf einen Träger aufgebracht werden. Zur Vorpolymerisation wird bevorzugt das (oder eines der) in der Polymerisation eingesetzte(n) Olefin(e) verwendet.

Geeignete Träger sind beispielsweise Silikagele, Aluminiumoxide, festes Aluminoxan oder andere anorganische Trägermaterialien. Ein geeignetes Trägermaterial ist auch ein Polymerpulver in feinverteilter Form.

Erfindungsgemäß können an Stelle oder neben eines Aluminoxans Verbindungen der Formeln RₓNH₄₋ₓBR'₄, RₓPH₄₋ₓBR'₄, R₃CBR'₄ oder BR'₃ als geeignete Cokatalysatoren verwendet werden. In diesen Formeln bedeutet x eine Zahl von 1-4, bevorzugt 3, die Reste R sind gleich oder verschieden, bevorzugt gleich, und bedeuten C₁-C₁₀-Alkyl, C₆-C₁₈-Aryl oder 2 Reste R bilden zusammen mit dem sie verbindenden Atom einen Ring, und die Reste R' sind gleich oder verschieden, bevorzugt gleich, und stehen für C₆-C₁₈-Aryl, das durch Alkyl, Haloalkyl oder Fluor substituiert sein kann.

Insbesondere steht R für Ethyl, Propyl, Butyl oder Phenyl und R' für Phenyl, Pentafluorphenyl, 3,5-Bistrifluormethylphenyl, Mesityl, Xylyl oder Tolyl (vgl. EP-A 277 003, EP-A 277 004 und EP-A 426 638).

Bei Verwendung der obengenannten Cokatalysatoren besteht der eigentliche (aktive) Polymerisationskatalysator aus dem Reaktionsprodukt von Metallocen und einer der genannten Verbindungen. Daher wird zunächst dieses Reaktionsprodukt bevorzugt außerhalb des Polymerisationsreaktors in einem separaten Schritt unter Verwendung eines geeigneten Lösungsmittels hergestellt (vgl. Ausführungsbeispiel VIII).

Prinzipiell ist als Cokatalysator erfindungsgemäß jede Verbindung geeignet, die aufgrund ihrer Lewis-Acidität das neutrale Metallocen in ein Kation überführen und dieses stabilisieren kann ("labile Koordination"). Darüberhinaus soll der Cokatalysator bzw. das aus ihm gebildete Anion keine weiteren Reaktionen mit dem gebildeten Metallocenkation eingehen (vgl. EP-A 427 697).

Zur Entfernung von im Olefin vorhandener Katalysatorgifte ist eine Reinigung mit einem Aluminiumalkyl, beispielsweise AlMe₃ oder AlEt₃ vorteilhaft. Diese Reinigung kann sowohl im Polymerisationssystem selbst erfolgen, oder das Olefin wird vor der Zugabe in das Polymerisationssystem mit der Al-Verbindung in Kontakt gebracht und anschließend wieder abgetrennt.

Die Polymerisation oder Copolymerisation wird in bekannter Weise in Lösung, in Suspension oder in der Gasphase kontinuierlich oder diskontinuierlich, ein- oder mehrstufig bei einer Temperatur von -60 bis 200°C, vorzugsweise 30 bis 80°C, durchgeführt. Polymerisiert oder copolymerisiert werden Olefine der Formel R^{a}-CH=CH-R^{b}. In dieser Formel sind R^{a} und R^{b} gleich oder verschieden und bedeuten ein Wasserstoffatom oder einen Alkylrest mit 1 bis 14 C-Atomen. R^{a} und R^{b} können jedoch auch mit den sie verbindenen C-Atomen einen Ring bilden. Beispiele für solche Olefine sind Ethylen, Propylen, 1-Buten, 1-Hexen, 4-Methyl-1-penten, 1-Octen, Norbornen oder Norbornadien. Insbesondere werden Propylen und Ethylen polymerisiert.

Als Molmassenregler wird, falls erforderlich, Wasserstoff zugegeben. Der Gesamtdruck im Polymerisationssystem beträgt 0,5 bis 100 bar. Bevorzugt ist die Polymerisation in dem technisch besonders interessanten Druckbereich von 5 bis 64 bar.

Dabei wird das Metallocen in einer Konzentration, bezogen auf das Übergangsmetall, von 10⁻³ bis 10⁻⁸, vorzugsweise 10⁻⁴ bis 10⁻⁷ mol Übergangsmetall pro dm³ Lösemittel bzw. pro dm³ Reaktorvolumen angewendet. Das Aluminoxan wird in einer Konzentration von 10⁻⁵ bis 10⁻¹ mol, vorzugsweise 10⁻⁴ bis 10⁻² mol pro dm³ Lösemittel bzw. pro dm³ Reaktorvolumen verwendet. Die anderen genannten Cokatalysatoren werden in etwa äquimolaren Mengen zum Metallocen verwendet. Prinzipiell sind aber auch höhere Konzentrationen möglich.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Pentan, Hexan, Heptan, Isooctan, Cyclohexan, Methylcyclohexan genannt.

Weiterhin kann eine Benzin- bzw. hydrierte Dieselölfraktion benutzt werden. Brauchbar ist auch Toluol. Bevorzugt wird im flüssigen Monomeren polymerisiert.

Werden inerte Lösemittel verwendet, werden die Monomeren gasförmig oder flüssig zudosiert.

Die Dauer der Polymerisation ist beliebig, da das erfindungsgemäß zu verwendende Katalysatorsystem einen nur geringen zeitabhängigen Abfall der Polymerisationsaktivität zeigt.

Das erfindungsgemäße Verfahren zeichnet sich dadurch aus, daß die beschriebenen Metallocen-Katalysatorsysteme im technisch interessanten Temperaturbereich zwischen 30 und 80°C, insbesondere aber im Bereich zwischen 60 und 80°C, Polymere mit enger Molmassenverteilung und grobkörniger Kornmorphologie sowie variabler Molmasse und Stereotaktizität erzeugen. Die jeweils gewünschte Polymermolmasse und Stereotaktizität wird durch Wahl der geeigneten Substituenten in den 2- und 4-Stellungen des Ligandsystems des Metallocens eingestellt. Wird ohne Wasserstoff als Molmassenregler polymerisiert, weisen die Polymeren ungesättigte Endgruppen auf.

Die nachfolgenden Beispiele sollen die Erfindung näher erläutern.

Es bedeuten:
- VZ =: Viskositätszahl in cm³/g 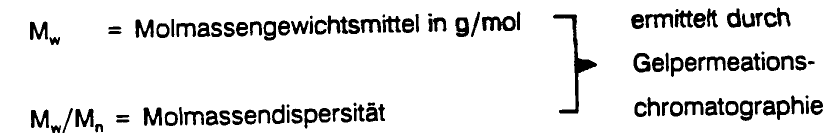
- Schmp. =: Schmelzpunkt ermittelt mit DSC (20°C/min Aufheiz-/ Abkühlgeschwindigkeit)
- II =: Isotaktischer Index (II = mm+1/2 mr) ermittelt durch ¹³C-NMR-Spektroskopie
- mmmm =: Anteil isotaktischer Pentaden im ¹³C-NMR-Spektrum in Prozent.
- SD =: Polymerschüttdichte in g/cm³
- d₅₀ =: mittlerer Polymerkorndurchmesser in µm
- MFI/(230/5) =: Schmelzindex, gemessen nach DIN 53735; in g/10 min

### Synthese der in den Beispielen verwendeten Metallocene:

### I) Metallocen A: rac-Dimethylsilylenbis(1-(2-methyl-4-ethylindenyl))zirkondichlorid

### I.1. 4-Ethylinden (a2)

20,7 g (181,7 mmol) 4-Oxocapronaldehyd (a1, hergestellt aus Propionsäurechlorid und Allylchlorid; vgl. Synthesis, (1985) 1058) wurden in 10 ml abs. Methanol gelöst und unter Kühlung mit einer Lösung von 13,2 g (199 mmol) Cyclopentadien in 5 ml abs. Methanol versetzt. Diese Mischung wurde bei 0°C innerhalb 35 min. zu einer Lösung von 51 g (454 mmol) Kaliumtertiärbutylat in 100 ml abs. Methanol getropft, wobei eine Dunkelbraunfärbung auftrat. Nach 2-4 h Rühren bei 0°C und weiteren 2 h bei Raumtemperatur wurde die Mischung auf Eis gegossen, auf pH 6 eingestellt und mit Methylenchlorid extrahiert. Die organische Phase wurde mit gesättigter NaCl-Lösung gewaschen, über Natriumsulfat getrocknet und eingedampft. Das Rohprodukt wurde an 750 g Kieselgel 60 chromatographiert. Mit Hexan/Methylenchlorid (20:1 bis 10:1) ließen sich 11,1 g (43 %) des Indens a2 isolieren (2 Doppelbindungsisomere 3:2).

### I.2. 4-Ethyl-2-indanon (a3)

33,9 g (235 mmol) 4-Ethylinden (a2) wurden unter Eiskühlung langsam zu einer Mischung von 141 ml Ameisensäure (98-100 %) und 33 ml (340 mmol) H₂O₂ (35 %) getropft (stark exotherme Reaktion). Anschließend wurde noch 2,5 h bei Raumtemperatur gerührt. Die entstehende gelborange Suspension wurde im Wasserstrahlvakuum von überschüssiger Ameisensäure befreit. Das zurückgebliebene gelbe Öl wurde mit 900 ml 2N Schwefelsäure versetzt. Unter Nachdosieren von Wasser wurden insgesamt 3 l Wasser überdestilliert, wobei sich das Produkt als gelbliches Öl in der Vorlage abschied. Das Destillat wurde mit gesättigter Natriumcarbonatlösung neutralisiert und ausgeethert. Die Etherphase wurde über Natriumsulfat getrocknet und eingedampft. Man erhielt 22,4 g (59 %) der Verbindung a3 als weißen Feststoff.

### I.3. 2-Methyl-4-ethylinden (a4)

Eine Lösung von 22,4 g (140 mmol) a3 in 500 ml Diethylether wurde bei Raumtemperatur unter Ar-Schutz innerhalb 1 h mit 140 ml (420 mmol) einer 3 M etherischen Methylmagnesiumbromid-Lösung versetzt. Anschließend wurde noch 2 h bei Raumtemperatur unter Rückfluß und weitere 15 h bei Raumtemperatur gerührt. Die Mischung wurde auf HCl-saures Eis gegossen und ausgeethert. Nach dem Trocknen über Natriumsulfat wurde das Lösemittel abgezogen. Das zurückgebliebene gelbe Öl (20,3 g) wurde in 800 ml Toluol p.a. aufgenommen, mit 2,2 g (11,5 mmol) p-Toluolsulfonsäurehydrat versetzt und 45 min refluxiert. Nach dem Abkühlen wurde die Lösung mehrmals mit Wasser gewaschen, über Natriumsulfat getrocknet und eingedampft. Der Rückstand wurde an 620 g Kieselgel 60 chromatographiert. Mit Hexan/Methylenchlorid (20:1) ließen sich 5,5 g (25 %) des Indens a4 eluieren (gelbliches Öl). Mit Hexan/Essigester (9:1) konnte noch unverbrauchtes Edukt a3 zurückgewonnen werden.

### I.4. Dimethylsilylenbis(2-methyl-4-ethylinden) (a5)

Eine Lösung von 5,5 g (34,8 mmol) a4 in 30 ml THF wurde unter Ar-Schutz bei 0°C langsam mit 14 ml (34,8 mmol) einer 2,5 M n-Butyilithium-Lösung in Hexan versetzt und anschließend 2 h unter Rückfluß erhitzt. Die dunkelbraune Lösung wurde anschließend langsam zu einer Lösung von 2,2 g (17,4 ml) Dimethyldichlorsilan in 15 ml THF getropft. Die Mischung wurde insgesamt 10 h unter Rückfluß erhitzt und über Nacht bei Raumtemperatur gerührt, anschließend wurde auf Eis gegossen und mit Diethylether extrahiert. Der nach Abziehen des Lösemittels verbliebene Rückstand wurde an 200 g Kieselgel chromatographiert. Mit Hexan/Methylenchlorid (20:1 bis 10:1) wurden zunächst 2,0 g unverbrauchtes Edukt a4 eluiert. Anschließend folgten 3,1 g des Produktes a5 (48 % Ausbeute bzgl. Si, 75 % bzgl. umges. Ed.). Die Verbindung fällt als gelbliches Öl an (2 Isomere 3:1).

### I.5. rac-Dimethylsilylenbis{1-(2-methyl-4-ethylindenyl)}-zirkondichlorid (A)

Eine Lösung von 3,1 g (8,3 mmol) des Ligandsystems a5 in 30 ml Diethylether wurde bei Raumtemperatur unter Ar-Schutz mit 10 ml (25 mmol) einer 2,5 M Butyllithium-Lösung in Hexan versetzt. Zunächst trat eine Orangefärbung und nach 45 min eine Eintrübung der Lösung ein. Nach dem Rühren über Nacht wurde die nunmehr beigefarbene Suspension mit 10 ml Hexan versetzt und über eine G3-Fritte filtriert. Der Niederschlag wurde mit 20 ml Hexan gewaschen und lange im Ölpumpenvakuum getrocknet. Das nahezu farblose Pulver wurde bei -78°C rasch zu einer Suspension von 1,8 g (7,72 mmol) Zirkontetrachlorid in 30 ml Methylenchlorid gegeben. Die Mischung wurde in 1-2 h auf Raumtemperatur erwärmt und nach 30 min Rühren bei Raumtemperatur vollständig eingedampft. Der im Ölpumpenvakuum getrocknete Rückstand wurde zunächst mit 60 ml Hexan gewaschen. Das Produkt wurde dann durch mehrmaliges Extrahieren mit insgesamt 180 ml Toluol gewonnen. Die vereinigten Extrakte wurden eingeengt und bei -35°C der Kristallisation überlassen. Die 1. Fraktion lieferte 0,76 g des Zirkonocens A in der reinen racemischen Form (orangefarbene Kristalle).Die weiteren Fraktionen enthielten einen steigenden Anteil an der meso-Form. Insgesamt wurden 1,78 g (43 %) der Verbindung A gewonnen. ¹H-NMR (CDCl₃) des Racemats: 6,85-7,55 (m,6,Aromaten-H), 6,80 (s,2,β-H), 2,72 (q,4,CH₂), 2,20 (s,6,CH₃), 1,30 (t,6,CH₃), 1,27 (s,6,Si-CH₃),
¹H-NMR (CDCl₃) der meso-Form: 6,6-7,6 (m,6,Arom.-H), 6,68 (s,2,β-H), 2,7 (q,4,CH₂), 2,48 (s,6,CH₃), 1,13-1,43 (m,12,Et-CH₃,Si-CH₃).

### II. Metallocen B: rac-Dimethylsilylenbis{1-(2-methyl-4-isopropylindenyl)}-zirkondichlorid

### II.1 4-Isopropylinden (b2)

Durch Umsetzung von iso-Buttersäurechlorid und Allylchlorid wurde 5-Methyl-4-oxocapronaldehyd (b1) analog zu a1 hergestellt (s. I.1.). 45,6 g (356 mmol) b1 wurden analog Vorschrift I.1. mit Cyclopentadien und Kaliumtertiärbutylat umgesetzt und aufgearbeitet. Die Säulenchromatographie lieferte 19,6 g (35 %) des Indens b2 als gelbes Öl (2 Doppelbindungsisomere).

### II.2. 4-Isopropyl-2-indanon (b3)

33,8 g (213 mmol) der Verbindung b2 wurden analog Vorschrift I.2. oxidiert und mit Wasser destilliert. Man erhielt 22,6 g (61 %) des Indanons b3 als gelblichen Feststoff.

### II.3. 2-Methyl-4-isopropylinden (b4)

11,1 g (63,8 mmol) des Indanons b3 wurden mit 2,5 eq. Methylmagnesiumbromid analog Vorschrift I.3. umgesetzt. Die Reaktionszeit betrug 17 h bei Raumtemperatur. Anschließend wurde mit p-Toluolsulfonsäurehydrat 25 min refluxiert. Die Chromatographie lieferte 3,9 g (36 %) des Indens b4 farbloses Öl.

### II.4. Dimethylsilylenbis(2-methyl-4-isopropylinden) (b5)

3,9 g (22,7 mmol) des Indens b4 wurden analog Vorschrift I.4. mit Dimethyldichlorsilan umgesetzt und aufgearbeitet. Die Säulenchromatographie lieferte neben 0,44 g unverbrauchtem Inden 3,0 g des Produktes b5 als gelbes Öl (Isomere). Die Ausbeute betrug 65 % bzgl. Si und 73 % bzgl. umges. Ed.

### II.5. rac-Dimethylsilylenbis{1-(2-methyl-4-isopropylindenyl)}zirkondichlorid (B)

3,0 g des Ligandsystems b5 wurden analog Vorschrift I.5. deprotoniert und mit 1 eq. Zirkontetrachlorid in 20 ml Methylenchlorid umgesetzt. Nach dem Waschen des Rohproduktes mit 40 ml Hexan wurde das Produkt mit insgesamt 120 ml Toluol extrahiert. Der Toluol-Extrakt wurde im Ölpumpenvakuum eingedampft. Man erhielt 1,7 g (46 %) des Zirkonocens als orangefarbenes Pulver. Das Racemat und die meso- Form lagen im Verhältnis 1:1 vor. Durch Umkristallisation aus wenig Toluol oder aus Toluol/Hexan-Mischungen ließ sich die racemische Form rein isolieren.
¹H-NMR des Racemats (CDCl₃): 6,7-7,5 (m,6,Arom.-H), 6,85 (s,2,β-H), 3,0 (m,2,i-Pr-CH) 2,23 (s,6,CH₃) 1,17-1,37 (d,12,i-Pr-CH₃) 1,27 (s,6,Si-CH₃).
¹H-NMR der meso-Form (CDCl₃): 6,5-7,5 (m,6,Arom.-H) 6,75 (s,2,β-H) 3,0 (m,2,i-Pr-CH) 2,48 (s,6,CH₃) 1,10-1,45 (m,18,i-Pr-CH₃,Si-CH₃).

### III. Metallocen C: rac-Dimethylsilylenbis{1-(2-methyl-4-tertiärbutylindenyl)}zirkondichlorid

### III.1. 4-Tertiärbutylinden (c2)

Durch Umsetzung von Pivalinsäurechlorid und Allylchlorid wurde 5,5-Dimethyl-4-oxocapronaldehyd c1 analog zu a1 hergestellt (s. I.1.). 41 g (195 mmol) c1 wurden analog Vorschrift I.1. mit Cyclopentadien und Kaliumtertiärbutylat umgesetzt und aufgearbeitet. Die Reaktionszeit betrug 19 h bei Raumtemperatur. Die Säulenchromatographie lieferte 3,2 g (10 %) des Indens c2 als gelbes Öl (2 Doppelbindungsisomere).

### III.2. 4-Tertiärbutyl-2-indanon (c3)

8,5 g (49,4 mmol) der Verbindung c2 wurden analog Vorschrift I.2. oxidiert und mit Wasser destilliert. Die Reaktionszeit betrug 4 h bei Raumtemperatur. Man erhielt 2,8 g (30 %) des Indanons c3 in Form gelber Kristalle.

### III.3. 2-Methyl-4-tertiärbutylinden (c4)

3,6 g (19 mmol) des Indanons c3 wurden mit 3,0 eq. Methylmagnesiumbromid analog Vorschrift I.3. umgesetzt und aufgearbeitet. Die Reaktionszeit betrug 17 h bei Raumtemperatur und weitere 4 h unter Rückfluß. Anschließend wurde mit p-Toluolsulfonsäurehydrat 25 min refluxiert. Die Chromatographie lieferte 1,2 g (33 %) des Indens c4 als gelbes Öl. Mit Hexan/Essigester (9:1) konnte unverbrauchtes Edukt zurückgewonnen werden.

### III.4. Dimethylsilylenbis(2-methyl-4-tertiärbutylinden) (c5)

1,2 g (6,4 mmol) des Indens c4 wurden analog Vorschrift I.4. mit Dimethyldichlorsilan umgesetzt und aufgearbeitet. Die Reaktionszeit betrug 10 h bei Rückfluß und 3 Tage bei Raumtemperatur. Die Säulenchromatographie lieferte neben 0,48 g unverbrauchtem Inden c4 0,40 g des Produktes c5 als gelbes Öl (Isomere). Die Ausbeute betrug 29 % bzgl. Si und 49 % bzgl. umgesetztes Edukt c4.

### III.5. rac-Dimethylsilylenbis{1-(2-methyl-4-tertiärbutylindenyl)} zirkondichlorid (C)

0,40 g (0,93 mmol) des Ligandsystems c5 wurden in 9 ml Diethylether unter Ar-Schutz mit 0,74 ml (1,86 mmol) einer 2,5 M n-Butyllithium-Lösung in Hexan versetzt. Nach Rühren über Nacht wurde die orange Lösung vollständig eingedampft. Der Rückstand wurde lange im Ölpumpenvakuum getrocknet und rasch bei -78°C zu einer Suspension von 225 mg (0,96 mmol) Zirkontetrachlorid in 5 ml Methylenchlorid gegeben. Die Mischung wurde 2 h bei 0°C und 30 min. bei Raumtemperatur gerührt und vollständig eingedampft. Das Produkt wurde mit insgesamt 8 ml Toluol extrahiert. Nach Abziehen des Toluols erhielt man 210 mg (37 %) des Zirkonocens als oranges Pulver. Das Verhältnis von Racemat zur meso-Form betrug 1:1. Durch Umkristallisation aus Toluol/Hexan konnte die reine racemische Form gewonnen werden. ¹H-NMR des Racemats (CDCl₃): 6,8-7,5 (m,6,Arom.-H) 6,92 (s,2,β-H) 2,27 (s,6,CH₃) 1,22-1,41 (m,24,t-Bu,Si-CH₃).
¹H-NMR der meso-Form (CDCl₃): 6,7-7,6 (m,6,Arom.-H) 6,7 (s,2,β-H) 2,50 (s,6,CH₃) 1,1-1,5 (m,24,t-Bu,Si-CH₃).

### IV. Metallocen D: rac-Methylphenylsilylenbis{1-(2-methyl-4-isopropylindenyl)}-zirkondichlorid

### IV.1. Methylphenylsilylenbis(2-methyl-4-isopropylinden) (d5)

Eine Lösung von 2,0 g (11,8 mmol) 2-Methyl-4-isopropylinden b4 (s. I1.3.) in 40 ml THF wurde unter Ar-Schutz bei 0°C mit 4,8 ml einer 2,5 M Butyllithium-Lösung in Hexan versetzt und 90 min zum Rückfluß erhitzt. Anschließend wurde die rote Lösung zu einer Lösung von 1,12 g (5,9 mmol) Methylphenyldichlorsilan in 15 ml THF gegeben und 7 h unter Rückfluß erhitzt. Die Mischung wurde auf Eis gegossen und ausgeethert. Die über Natriumsulfat getrocknete Etherphase wurde im Vakuum eingedampft. Der verbleibende Rückstand wurde an 200 g Kieselgel 60 chromatographiert. Mit einem Laufmittelgemisch aus Hexan/Methylenchlorid (10:1) wurden zunächst 0,57 g unverbrauchtes Inden b4 zurückgewonnen. Mit Hexan/ Methylenchlorid (10:2) folgten 1,2 g des Produktes d5. Die Ausbeute betrug 44 % bzgl. Si und 61 % bzgl. umges. Inden b4.

### IV.2. rac-Methylphenylsilylenbis{1-(2-methyl-4-isopropylindenyl)}-zirkondichlorid (D)

Eine Lösung von 1,28 g (2,76 mmol) des Ligandsystems d5 in 20 ml Diethylether wurde bei Raumtemperatur unter Ar-Schutz langsam mit 3,3 ml (8,3 mmol) einer 2,5 M Butyllithium-Lösung in Hexan versetzt und über Nacht gerührt. Die orangefarbene Lösung wurde vollständig eingedampft, lange im Ölpumpenvakuum getrocknet und mit insgesamt 20 ml Hexan gewaschen. Der Rückstand wurde lange im Ölpumpenvakuum bei 40°C getrocknet und pulverisiert. Das gelbe Pulver wurde bei -78°C zu einer Suspension von 0,62 g (2,66 mmol) Zirkontetrachlorid in 15 ml Methylenchlorid gegeben. Die Mischung wurde in 1 h auf 0°C aufgewärmt und weitere 2 h bei Raumtemperatur gerührt. Die rotbraune Suspension wurde vollständig eingedampft und im Ölpumpenvakuum getrocknet. Mit Toluol wurden 1,05 g (63 %) des Zirkonocens extrahiert (oranges Pulver). Im Rohprodukt lagen 1 racemische und 2 meso-Formen im Verhältnis 2:1:1 vor. Durch Umkristallisation aus Toluol/Hexan konnte die racemische Form isoliert werden.
¹H-NMR der Isomerenmischung (CDCl₃): 6,4-8,2 (m,Arom,-H,β-H) 3,1 (br,i-Pr-CH) 2,55 (s,CH₃) 2,33 (s,CH₃) 2,22 (s,CH₃) 1,95 (s,CH₃) 1,13-1,47 (m,i-Pr-CH₃,Si-CH₃).

### V. Metallocen E: rac-Dimethylsilylenbis{1-(2-ethyl-4-methylindenyl)}zirkondichlorid

### V.1. 2-(2-Methylbenzyl)-buttersäure (e1)

14,2 g (0,62 mol) Natrium wurden in 250 ml Ethanol gelöst und 118,4 g (0,63 mol) Ethyl-diethylmalonester zugegeben. Es wurden 118,5 g (0,64 mol) 2-Methylbenzylbromid so zugetropft, daß die Mischung leicht siedete. Anschließend wurde 4 h unter Rückfluß erhitzt. Die Suspension wurde auf Wasser gegeben, mit Ether extrahiert und die vereinigten organischen Phasen wurden über MgSO₄ getrocknet. Das Lösungsmittel wurde i.V. entfernt und das erhaltene Rohprodukt (187 g) ohne weitere Reinigung weiter umgesetzt.

Zur Verseifung wurde in Gegenwart von 139 g KOH in 355 ml Ethanol und 170 ml H₂O 15 h unter Rückfluß erhitzt. Das Lösungsgemisch wurde i.V. abgezogen und der Rückstand unter Kühlung mit konz. Salzsäure bis zum pH 1 versetzt. Es wurde 3mal mit Ether extrahiert, die vereinigten organischen Phasen wurden mit gesättigter wäßriger NaCl-Lösung gewaschen und über MgSO₄ getrocknet. Das Lösungsmittel wurde entfernt und der Rückstand zur Decarboxylierung auf 170°C erhitzt, wobei das Produkt e1 abdestillierte (140-145°C/0.1 Torr).
Ausbeute: 96,0 g (81 %).

### V.2. 2-(2-Methyl-benzyl)-buttersäurechlorid (e2)

96 g (0,5 mol) 2-(o-Xylyl)-buttersäure (e1) wurden langsam mit 89 g (0,75 mol) SOCl₂ erhitzt und bis zum Ende der Gasentwicklung (1 h) refluxiert. Überschüssiges Thionylchlorid wurde abdestilliert, und Reste durch dreimaliges Abziehen von je 50 ml Toluol i.V. entfernt. Das Rohprodukt wurde durch Destillation (103°C/1 Torr) gereinigt.
Ausbeute: 101,7 g (96 %, 0,48 mol).

### V.3. 2-Ethyl-4-methyl-1-indanon (e3)

101,7 g (0,48 mol) 2-(2-Methyl-benzyl)-buttersäurechlorid (e2) wurden zu 191 g (1,43 mol) AlCl₃ in 600 ml Toluol zugetropft und ca. 3,5 h auf 80°C erwärmt. Die Reaktionsmischung wurde auf 1 l Eis/konz. HCl gegossen und die Phasen wurden getrennt. Die wäßrige Phase wurde 4mal mit je 250 ml Toluol extrahiert, die vereinigten organischen Phasen mit gesättigter wäßriger NaHCO₃- und NaCl-Lösung gewaschen und über MgSO₄ getrocknet. Das Lösungsmittel wurde i.V. entfernt und der Rückstand destilliert (78°C/0,2 Torr).
Ausbeute: 81 g (97 %, 0,464 mol).

### V.4. 2-Ethyl-4-methyl-inden (e4)

34.1 g (196 mmol) 2-Ethyl-4-methyl-1-indanon (e3) wurden in 210 ml THF/Methanol (2:1) portionsweise mit 11,1 g (294 mmol) NaBH₄ versetzt und 15 h bei Raumtemperatur gerührt. Die Reaktionsmischung wurde auf Eis gegossen und mit konz. HCl bis pH = 1 versetzt. Nach Extraktion mit Ether wurden die vereinigten organischen Phasen mit gesättigter wäßriger NaHCO₃- und NaCl-Lösung gewaschen und über MgSO₄ getrocknet. Der i.V. vom Lösungsmittel befreite Rückstand (36,2 g) wurde für die folgende Eliminierung direkt weiter umgesetzt.

Das nicht gereinigte 2-Ethyl-4-methyl-1-indanol wurde in 700 ml Toluol in Gegenwart von 0,75 g p-Toluolsulfonsäure-monohydrat 2 h auf dem Dampfbad behandelt. Das Lösungsgemisch wurde i.V. entfernt, der Rückstand in Ether aufgenommen, mit gesättigter NaHCO₃- und NaCl-Lösung gewaschen und über MgSO₄ getrocknet. Das Lösungsmittel wurde i.V. entfernt und der Rückstand destilliert (62°C/ 0,2 Torr).
Ausbeute: 25,7 g (83 %, 162 mmol).

### V.5. Dimethylsilylenbis(2-ethyl-4-methylinden) (e5)

Zu 10,4 g (65,5 mmol) 2-Ethyl-4-methyl-inden (e4) in 50 ml abs. THF wurden 26,2 ml (65,6 mmol) einer 2,5 M BuLi-Lösung in Hexan langsam zugetropft und 2 h bei 50°C weitergerührt. Währenddessen legte man 3,95 ml Me₂SiCl₂ in 50 ml abs. THF vor und tropfte anschließend das Li-Salz im Verlauf von 8 h zu. Man rührte 15 h entfernte das Lösungsmittel i.V., suspendierte in n-Pentan und filtrierte erneut. Nach Entfernen des Lösungsgemisches reinigte man durch Säulenchromatographie über Silica- Gel (n-Hexan/CH₂Cl₂ 9:1).
Ausbeute:15,1 g (63 %, 41 mmol).

### V.6. rac-Dimethylsilylenbis{1-(2-ethyl-4-methylindenyl)}zirkondichlorid (E)

Zu 3,57 (9,58 mmol) Me₂Si(2-Et-4-Me-Ind)₂ in 50 ml THF tropfte man bei Raumtemperatur 7,66 ml (19,16 mmol) einer 2,5 M BuLi-Lösung in n-Hexan zu und erwärmte noch 3 h auf 50°C. Es wurde zur Trockene eingedampft, in n-Pentan suspendiert, filtriert und getrocknet. 2,23 (9,58 mmol) ZrCl₄ wurden in 150 ml CH₂Cl₂ suspendiert und auf -78°C gekühlt. Das Dilithio-Salz wurde zugegeben, 3 h bei -20°C gerührt, und über Nacht auf Raumtemperatur kommen lassen. Es wurde filtriert und das Lösungsmittel entfernt. Kristallisation aus Toluol/n-Hexan (25:1) ergab 0,18 g orange Kristalle (meso/rac 5:1). Die Mutterlauge wurde auf 1/4 ihres Volumens eingeengt und der Kristallisation bei -38°C überlassen, man erhielt weitere 0,1 g des Komplexgemisches. Die Mutterlauge wurde zur Trockene eingedampft, der Rückstand in n-Hexan suspendiert, filtriert und getrocknet. Man erhielt die reine rac-Form E als orangefarbenes Pulver.

### VI. Metallocen F: rac-Dimethylsilylenbis{1-(2-methyl-4-ethylindenyl)} zirkondimethyl

0,26 g Metallocen A in 40 cm³ Et₂O wurden bei -50°C tropfenweise mit 1,3 cm³ einer 1,6 M (2,08 mmol) etherischen MeLi-Lösung versetzt und 2 h bei -10°C gerührt. Nach Austausch des Lösemittels gegen n-Pentan wurde noch 1,5 h bei Raumtemperatur gerührt und der filtrierte Rückstand im Vakuum sublimiert. Es wurden 0,15 g Sublimat mit einer korrekten Elementaranalyse erhalten.

### VII. Umsetzung von Metallocen F mit [Bu₃NH][B(C₆H₅)₄]

0,15 g Metallocen F wurden bei 0°C zu 0,17 g [Bu₃NH][B(C₆H₅)₄] in 25 cm³ Toluol gegeben. Unter Rühren wurde auf 50°C erwärmt und die Mischung 10 min bei dieser Temperatur gerührt. Die tiefgefärbte Mischung wurde dann zur Trockne eingedampft. Für die Polymerisation wurde ein aliquoter Teil des Reaktionsgemisches verwendet (Bu = Butyl).
Abkürzungen: Me= Methyl, Et= Ethyl, Bu= Butyl, Ind= Indenyl.

### Polymerisationsbeispiele:

### Beispiel 1

Ein trockener 16-dm³-Reaktor wurde mit Stickstoff gespült und mit 10 dm³ flüssigem Propylen befüllt.

Dann wurden 30 cm³ toluolische Methylaluminoxanlösung (entsprechend 45 mmol Al, mittlerer Oligomerisierungsgrad n = 16) zugegeben und der Ansatz bei 30°C 15 Minuten gerührt.

Parallel dazu wurden 3,3 mg (0,006 mmol) Metallocen B in 20 cm³ toluolischer Methylaluminoxanlösung (30 mmol Al) gelöst und durch 15 minütiges Stehenlassen voraktiviert.

Die Lösung wurde dann in den Reaktor gegeben, durch Wärmezufuhr auf die Polymerisationstemperatur von 70°C aufgeheizt (4°C/min) und das Polymerisationssystem 1 h durch Kühlung bei 70°C gehalten. Gestoppt wurde die Polymerisation durch Zusatz von 20 ml Isopropanol. Das überschüssige Monomere wurde abgegast, das Polymer im Vakuum getrocknet. Man erhielt 1,44 kg Polypropylen.

Die Katalysatoraktivität betrug somit 436 kg PP/g Metallocen x h.
VZ = 168 cm³/g;
Schmp.= 149,6°C; II = 95 %;
mmmm = 88,6 %
SD = 0,30 g/cm³ ; d₅₀ = 2600 µm
M_{w} = 1,8·10⁵ g/mol
M_{w}/Mₙ = 2,2

### Beispiele 2-11

Es wurde jeweils analog Beispiel 1 verfahren, wobei jedoch folgende Größen variiert wurden:
- Art des Metallocens
   u- Menge des Metallocens (mg)
- Polymerisationstemperatur

Die variierten Polymerisationsparameter und die Polymerausbeute sind Tabelle 1, die an den Polymeren gemessenen Werte Tabelle 2 zu entnehmen.

**Tabelle 1**

| Beispiel | Metallocen | | Polymerisationstemperatur [°C] | Polymerausbeute [kg] |
|---|---|---|---|---|
| | Art | Menge [mg] | | |
| 2 | B | 3,1 | 60 | 0,82 |
| 3 | B | 6,4 | 50 | 1,30 |
| 4 | D | 10,1 | 30 | 0,75 |
| 5 | A | 4,4 | 70 | 1,10 |
| 6 | A | 6,3 | 50 | 0,55 |
| 7 | A | 6,1 | 30 | 0,25 |
| 8 | E | 3,0 | 70 | 0,50 |
| 9 | E | 2,7 | 50 | 0,26 |

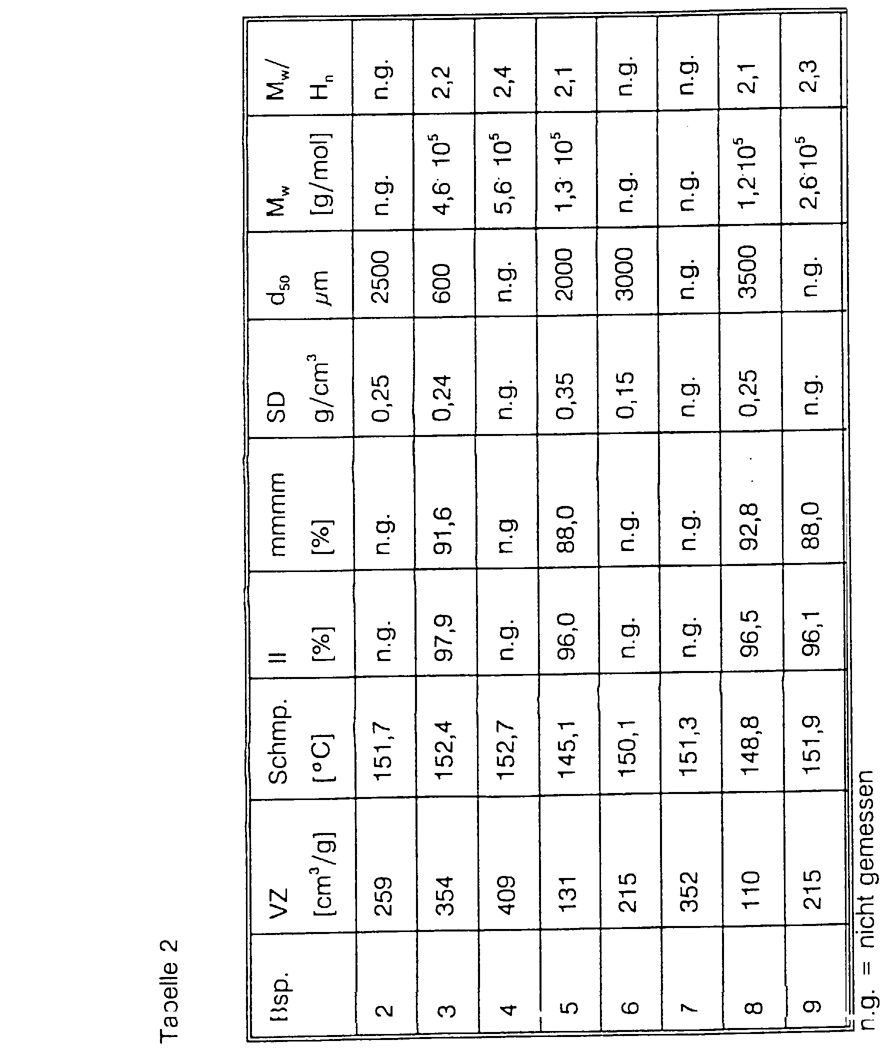

### Beispiel 10

Es wurde verfahren wie in Beispiel 1. Direkt nach Zugabe des Metallocens zum Reaktor wurden aber noch 0,3 bar (30.000 N/m²) Wasserstoff auf den Reaktor aufgedrückt.
Man erhielt 1,00 kg Polymer.
Vz = 76 cm³/g;
Schmp.: = 148,9°C;
SD = 0,20 g/cm³ ; d₅₀ = 1800 µm
M_{w} = 6,2·10⁴ g/mol
M_{w}/Mₙ = 2,8

### Beispiel 11

Ein trockener 16-dm³-Reaktor wurde mit Stickstoff gespült und mit 10 dm³ flüssigem Propylen befüllt.
Dann wurden 2,5 cm³ Reaktionsgemisch nach Bsp. VII (entsprechend 15 mg Metallocen F) in 20 cm³ Toluol gelöst und bei Umgebungstemperatur in den Reaktor gegeben. Der Reaktor wurde durch Wärmezufuhr auf die Polymerisationstemperatur von 70°C aufgeheizt (4°C/min) und das Polymerisationssystem 1 h durch Kühlung bei 70°C gehalten. Gestoppt wurde die Polymerisation durch Zusatz von 20 ml Isopropanol. Das überschüssige Monomere wurde abgegast, das Polymer im Vakuum getrocknet. Man erhielt 0,8 kg Polypropylen.
VR= 120 cm³/g, Schmp.: 144,8°C.

### Beispiel 12

Ein trockener 70-dm³-Reaktor wurde mit Stickstoff gespült und mit 40 dm³ flüssigem Propylen befüllt.
Dann wurden 180 cm³ toluolische Methylaluminoxanlösung (entsprechend 270 mmol Al, mittlerer Oligomerisierungsgrad n= 16) zugegeben und der Ansatz bei 30°C 15 Minuten gerührt. Anschließend wurden 35 g Ethylen eindosiert. Parallel dazu wurden 10,9 mg Metallocen A in 20 cm³ toluolischer Methylaluminoxanlösung (30 mmol Al) gelöst und durch 15 minütiges Stehenlassen voraktiviert.

Die Lösung wurde dann in den Reaktor gegeben, der Reaktor wurde durch Wärmezufuhr innerhalb von 10 min auf die Polymerisationstemperatur von 50°C aufgeheizt und dort unter Rühren 4 h gehalten. Während der genannten 4 h wurden kontinuierlich weitere 85 g Ethylen eindosiert. Die Polymerisation wurde dann durch Zusatz von 20 ml Isopropanol gestoppt, das überschüssige Monomere abgegast, das Polymere im Vakuum getrocknet. Man erhielt 3,5 kg statistisches Propylen-Ethylen-Copolymer mit 3,0 Gew.-% Ethylengehalt.
VZ = 226 cm³/g; M_{w} = 2,3·10⁵ g/mol; M_{w}/Mₙ = 1,9

### Beispiel 13

Ein trockener 16-dm³-Reaktor wurde mit Stickstoff gespült und bei 20°C mit 10 dm³ eines entaromatisierten Benzinschnittes mit dem Siedebereich 100 - 120°C gefüllt.

Dann wurde der Gasraum des Kessels durch 5-maliges Aufdrücken von 2 bar (200.000 N/m²) Ethylen und Entspannen stickstofffrei gespült.

Dann wurden 30 cm³ toluolische Methylaluminoxanlösung (entsprechend 45 mmol Al, Molmasse nach kryoskopischer Bestimmung 750 g/mol) zugegeben.

Unter Rühren wurde der Reaktorinhalt innerhalb von 15 Minuten auf 30°C aufgeheizt und durch Zugabe von Ethylen wurde bei 250 Upm Rührgeschwindigkeit der Gesamtdruck auf 5 bar (500.000 N/m²) eingestellt.

Parallel dazu wurden 2,3 mg Metallocen C in 20 cm³ toluolischer Methylaluminoxanlösung gelöst und durch 15 minütiges Stehenlassen voraktiviert. Dann wurde die Lösung in den Reaktor gegeben, das Polymerisationssystem wurde auf eine Temperatur von 70°C gebracht und durch entsprechende Kühlung 1 h bei dieser Temperatur gehalten. Der Gesamtdruck wurde während dieser Zeit durch entsprechende Zufuhr von Ethylen bei 5 bar (500.000 N/m²) gehalten.

Die Polymerisation wurde durch Zugabe von 20 ml Isopropanol gestoppt, das Polymere abfiltriert und im Vakuum getrocknet.
Man erhielt 1,3 kg Polyethylen.
VZ = 542 cm³/g

## Patentansprüche (Patentansprüche für folgende(n) Vertragsstaat(en): AT, BE, CH, LI, DE, FR, GB, IT, LU, NL, SE)

1. Metallocen der Formel I 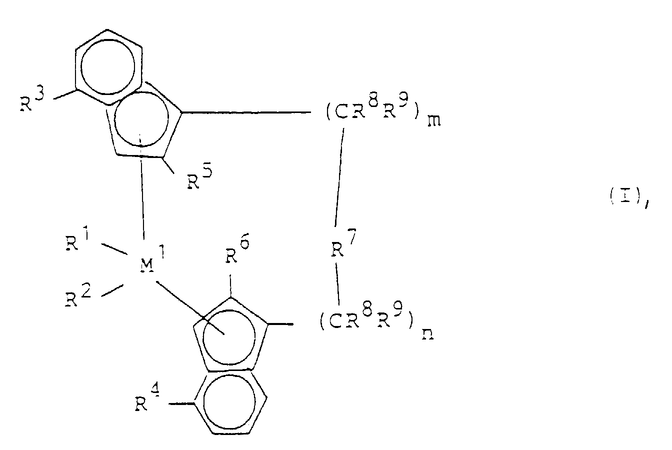 worin
M¹ ein Metall der Gruppe IVb, Vb oder Vlb des Periodensystems ist,
R¹ und R² gleich oder verschieden sind und Wasserstoff, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
R³ und R⁴ gleich oder verschieden sind und ein Halogenatom, eine C₁-C₁₀-Alkylgruppe, die halogeniert sein kann, eine C₆-C₁₀-Arylgruppe, einen -NR₂¹⁰, -SR¹⁰, -OSiR₃¹⁰, SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
R⁵ und R⁶ gleich oder verschieden sind und ein Halogenatom, eine C₁-C₁₀-Alkylgruppe, die halogeniert sein kann, eine C₆-C₁₀-ArylgrUppe, einen -NR₂¹⁰, -SR¹⁰, -OSiR₃¹⁰, SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
R⁷ 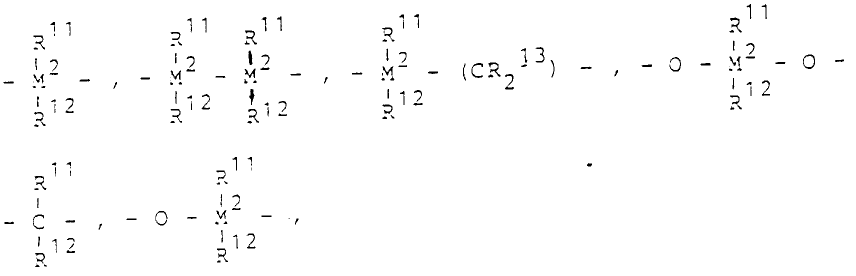 =BR¹¹, =AlR¹¹, -Ge-, -Sn-, -O-, -S-, =SO, =SO₂, =NR¹¹, =CO, =PR¹¹ oder =P(O)R¹¹ ist,
wobei
R¹¹, R¹² und R¹³ gleich oder verschieden sind und ein Wasserstoffatom, ein Halogenatom, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden, oder R¹¹ oder R¹² mit R⁸ oder R⁹ jeweils zusammen mit den sie verbindenden Atomen einen Ring bilden,
M² Silizium, Germanium oder Zinn ist,
R⁸ und R⁹ gleich oder verschieden sind und die für R¹¹ genannte Bedeutung haben und
m und n gleich oder verschieden sind und null, 1 oder 2 sind, wobei m plus n null, 1 oder 2 ist, und das Metallocen der Formel I verschieden von Dimethylsilylenbis (2,4-dimethylindenyl)zirkoniumdichlorid und Ethylenbis (2-methyl-4-methylindenyl) hafniumdichlorid ist.

2. Metallocen gemäß Anspruch 1, worin in Formel I M¹ Zr, R¹ und R² gleich oder verschieden sind und Methyl oder Chlor, R³ und R⁴ gleich oder verschieden sind und Methyl, Ethyl, n-Propyl, i-Propyl, n-Butyl, i-Butyl, tert.-Butyl oder Neopentyl, R⁵ und R⁶ gleich oder verschieden sind und Methyl oder Ethyl, R⁷ einen Rest 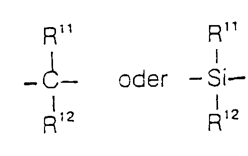 und n plus m null oder 1 bedeuten.

3. Metallocen gemäß einem oder mehreren der Ansprüche 1 oder 2, dadurch gekennzeichnet, daß in Formel I die Substituenten R¹ und R², R³ und R⁴ sowie R⁵ und R⁶ jeweils gleich sind.

4. rac-Dimethylsilylenbis(1-(2-methyl-4-ethylindenyl))zirkondichlorid, rac-Dimethylsilylenbis(1-(2-methyl-4-isopropylindenyl))-zirkondichlorid, rac-Dimethylsilylenbis (1-(2-methyl-4-tertiärbutylindenyl))-zirkondichlorid, rac-Methylphenylsilylenbis(1-(2-methyl-4-isopropylindenyl))zirkondichlorid, rac-Dimethylsilylenbis( 1 -(2-ethyl-4-methylindenyl))zirkondichlorid, rac-Dimethylsilylenbis(1-(2-methyl-4-ethylindenyl) zirkondimethyl.

5. Katalysator, welcher aus einem Metallocen der Formel I, gemäß einem oder mehreren der Ansprüche 1 bis 4, und einem Cokatalysator gebildet wird.

6. Katalysator gemäß Anspruch 5, worin der Cokatalysator ein Aluminoxan der Formel (II) 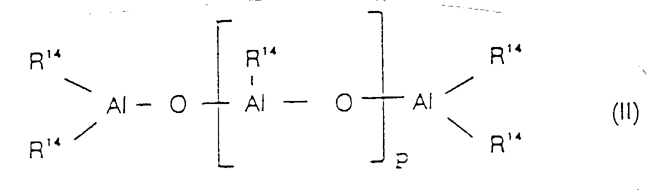 für den linearen Typ und/oder der Formel (III) 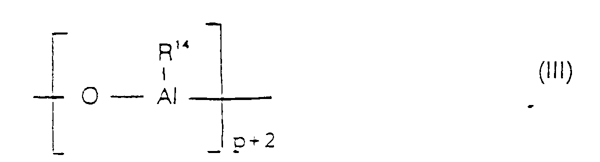 für den cyclischen Typ ist,
wobei in den Formeln (II) und (III) die Reste R¹⁴ gleich oder verschieden sind und eine C₁-C₆-Alkylgruppe, eine C₆-C₁₈-Arylgruppe oder Wasserstoff bedeuten und p eine ganze Zahl von 2 bis 50 ist.

7. Katalysator gemäß Anspruch 5 oder 6, worin der Cokatalysator Methylaluminoxan ist.

8. Katalysator gemäß einem oder mehreren der Ansprüche 5 bis 7, worin das Metallocen auf einen Träger aufgebracht ist.

9. Katalysator gemäß einem oder mehreren der Ansprüche 5 bis 8, worin das Metallocen vorpolymerisiert ist.

10. Verfahren zur Herstellung eines Olefinpolymers durch Polymerisation oder Copolymerisation eines Olefins der Formel R^{a}-CH=CH-R^{b}, worin R^{a} und R^{b} gleich oder verschieden sind und ein Wasserstoffatom oder einen Kohlenwasserstoffrest mit 1 bis 14 C-Atomen bedeuten, oder R^{a} und R^{b} bilden mit den sie verbindenden Atomen einen Ring, bei einer Temperatur von -60 bis 200°C, bei einem Druck von 0,5 bis 100 bar, in Lösung, in Suspension oder in der Gasphase, in Gegenwart eines Katalysators, gemäß einem oder mehreren der Ansprüche 5 bis 9.

11. Verfahren gemäß Anspruch 10, worin Ethylen oder Propylen polymerisiert werden.

12. Verfahren gemäß Anspruch 10 oder 11, worin das Metallocen der Formel I vor dem Einsatz in der Polymerisationsreaktion mit einem Aluminoxan der Formel II und/oder III voraktiviert wird.

## Patentansprüche (Patentansprüche für folgende(n) Vertragsstaat(en): ES)

1. Verfahren zur Herstellung eines Metallocens der Formel I 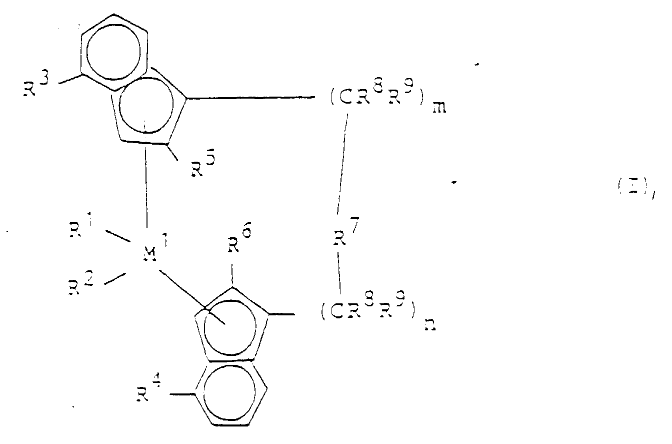 worin
M¹ ein Metall der Gruppe IVb, Vb oder VIb des Periodensystems ist,
R¹ und R² gleich oder verschieden sind und Wasserstoff, eine C₁-C₁₀-Alkylgruppe, eine C₁-C₁₀-Alkoxygruppe, eine C₅-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₃-C₄₀-Arylalkenylgruppe oder ein Halogenatom bedeuten,
R³ und R⁴ gleich oder verschieden sind und ein Halogenatom, eine C₁-C₁₀-Alkylgruppe, die halogeniert sein kann, eine C₆-C₁₀-Arylgruppe, einen -NR₂¹⁰, -SR¹⁰, -OSiR₃¹⁰, SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
R⁵ und R⁶ gleich oder verschieden sind und ein Halogenatom eine C₁-C₁₀-Alkylgruppe, die halogeniert sein kann, eine C₆-C₁₀-Arylgruppe, einen -NR₂¹⁰, -SR¹⁰, -OSiR₃¹⁰, SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
R⁷ 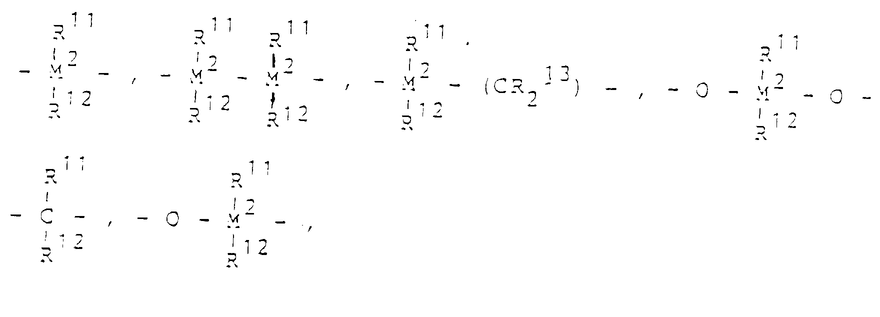 =BR¹¹, =AlR¹¹, -Ge-, -Sn-, -O-, -S-, =SO, =SO₂, =NR¹¹, =CO, =PR¹¹ oder =P(O)R¹¹ ist,
wobei
R¹¹, R¹² und R¹³ gleich oder verschieden sind und ein Wasserstoffatom, ein Halogenatom, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden, oder R¹¹ oder R¹² mit R⁸ oder R⁹ jeweils zusammen mit den sie verbindenden Atomen einen Ring bilden,
M² Silizium, Germanium oder Zinn ist,
R⁸ und R⁹ gleich oder verschieden sind und die für R¹¹ genannte Bedeutung haben und
m und n gleich oder verschieden sind und null, 1 oder 2 sind wobei m plus n null, 1 oder 2 ist, und das Metallocen der Formel I verschieden von Dimethylsilylenbis (2,4-dimethylindenyl)zirkoniumdichlorid und Ethylenbis (2-methyl-4-methylindenyl) hafniumdichlorid ist.

2. Verfahren gemäß Anspruch 1, worin in Formel I M¹ Zr, R¹ und R² gleich oder verschieden sind und Methyl oder Chlor, R³ und R⁴ gleich oder verschieden sind und Methyl, Ethyl, n-Propyl, i-Propyl, n-Butyl, i-Butyl, tert.-Butyl oder Neopentyl, R⁵ und R⁶ gleich oder verschieden sind und Methyl oder Ethyl, R⁷ einen Rest 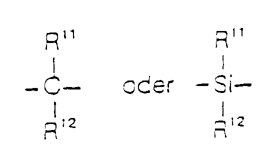 und n plus m null der 1 bedeuten.

3. Verfahren gemäß einem oder mehreren der Ansprüche 1 oder 2, dadurch gekennzeichnet, daß in Formel I die Substituenten R¹ und R², R³ und R⁴ sowie R⁵ und R⁶ jeweils gleich sind.

4. Verfahren gemäß einem oder mehreren der Ansprüche 1 bis 3, worin das Metallocen ausgewähtl ist von:
rac-Dimethylsilylenbis(1-(2-methyl-4-ethylindenyl))zirkondichlorid, rac-Dimethylsilylenbis(1-(2-methyl-4-isopropylindenyl))-zirkondichlorid, rac-Dimethylsilylenbis (1-(2-methyl-4-tertiärbutylindenyl))-zirkondichlorid, rac-Methylphenylsilylenbis(1-(2-methyl-4-isopropylindenyl))zirkondichlorid, rac-Dimethylsilylenbis(1-(2-ethyl-4-methyl-indenyl))zirkondichlorid, rac-Dimethylsilylenbis(1-(2-methyl-4-ethylindenyl)zirkondimethyl.

5. Verfahren zur Herstellung eines Katalysators, welcher aus einem Metallocen der Formel I, gemäß einem oder mehreren der Ansprüche 1 bis 4, und einem Cokatalysator gebildet wird.

6. Verharen gemäß Anspruch 5, worin der Cokatalysator ein Aluminoxan der Formel (II) 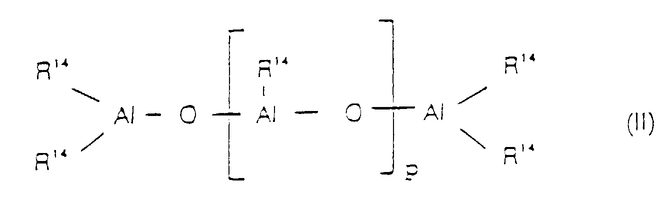 für den linearen Typ und/oder der Formel (III) 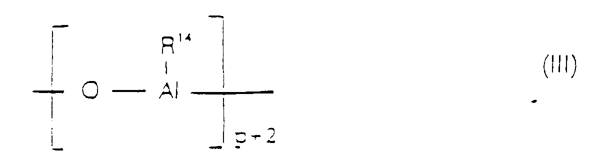 für den cyclischen Typ ist,
wobei in den Formeln (II) und (III) die Reste R¹⁴ gleich oder verschieden sind und eine C₁-C₆-Alkylgruppe, eine C₆-C₁₈-Arylgruppe cder Wasserstoff bedeuten und p eine ganze Zahl von 2 bis 50 ist.

7. Verfahren gemäß Anspruch 5 oder 6, worin der Cokatalysator Methylaluminoxan ist.

8. Verfahren gemäß einem oder mehreren der Ansprüche 5 bis 7, worin das Metallocen auf einen Träger aufgebracht ist.

9. Verfahren gemäß einem oder mehreren der Ansprücne 5 bis 8, worin das Metallocen vorpolymerisiert ist.

10. Verfahren zur Herstellung eines Olefinpolymers durch Polymerisation oder Copolymerisation eines Olefins der Formel R^{a}-CH=CH-R^{b}, worin R^{a} und R^{b} gleich oder verschieden sind und ein Wasserstoffatom oder einen Kohlenwasserstoffrest mit 1 bis 14 C-Atomen bedeuten, oder R^{a} und R^{b} bilden mit den sie verbindenden Atomen einen Ring, bei einer Temperatur von -60 bis 200°C, bei einem Druck von 0,5 bis 100 bar, in Lösung, in Suspension oder in der Gasphase, in Gegenwart eines Katalysators, gemäß einem oder mehreren der Ansprüche 5 bis 9.

11. Verfahren gemäß Anspruch 10, worin Ethylen oder Propylen polymerisiert werden.

12. Verfahren gemäß Anspruch 10 oder 11, worin das Metallocen der Formel I vor dem Einsatz in der Polymerisationsreaktion mit einem Aluminoxan der Formel II und/oder III voraktiviert wird.

## Claims (Claims for the following Contracting State(s): AT, BE, CH, LI, DE, FR, GB, IT, LU, NL, SE)

1. A metallocene of the formula I 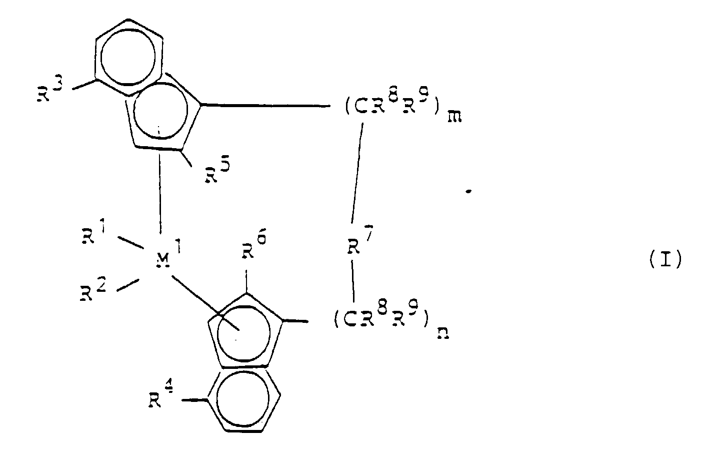 in which
M¹ is a metal of group IVb, Vb or VIb of the Periodic Table,
R¹ and R² are identical or different and are hydrogen, a C₁-C₁₀-alkyl group, a C₁-C₁₀-alkoxy group, a C₆-C₁₀-aryl group, a C₆-C₁₀-aryloxy group, a C₂-C₁₀-alkenyl group, a C₇-C₄₀-arylalkyl group, a C₇-C₄₀-alkylaryl group, a C₈-C₄₀-arylalkenyl group or a halogen atom,
R³ and R⁴ are identical or different and are a halogen atom, a C₁-C₁₀-alkyl group which may be halogenated, a C₆-C₁₀-aryl group or an -NR₂¹⁰, -SR¹⁰, -OSiR₃¹⁰, -SiR₃¹⁰ or -PR₂¹⁰ radical in which R¹⁰ is a halogen atom, a C₁-C₁₀-alkyl group or a C₆-C₁₀-aryl group,
R⁵ and R⁶ are identical or different and are a halogen atom, a C₁-C₁₀-alkyl group which may be halogenated, a C₆-C₁₀-aryl group or an -NR₂¹⁰, -SR¹⁰, -OSiR₃¹⁰, -SiR₃¹⁰ or -PR₂¹⁰ radical in which R¹⁰ is a halogen atom, a C₁-C₁₀-alkyl group or a C₆-C₁₀-aryl group,
R⁷ is 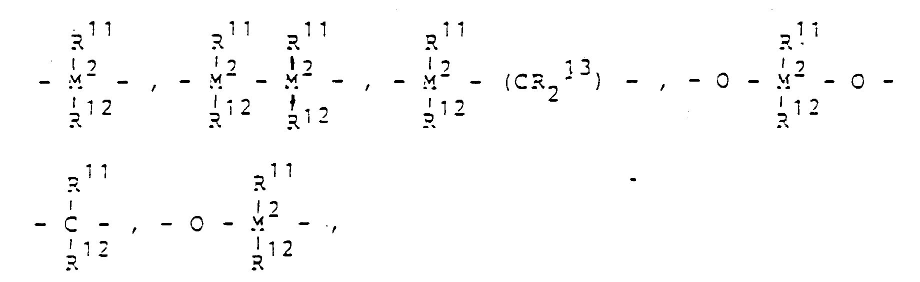 =BR¹¹, =AlR¹¹, -Ge-, -Sn-, -O-, -S-, =SO, =SO₂, =NR¹¹, =CO, =PR¹¹ or =P(O)R¹¹,
in which
R¹¹, R¹² and R¹³ are identical or different and are a hydrogen atom, a halogen atom, a C₁-C₁₀-alkyl group, a C₁-C₁₀-fluoroalkyl group, a C₆-C₁₀-aryl group, a C₆-C₁₀-fluoroaryl group, a C₁-C₁₀-alkoxy group, a C₂-C₁₀-alkenyl group, a C₇-C₄₀-arylalkyl group, a C₈-C₄₀-arylalkenyl group or a C₇-C₄₀-alkylaryl group, or R¹¹ and R¹² or R¹¹ and R¹³ in each case form a ring with the atoms joining them, or R¹¹ or R¹² with R⁸ or R⁹ in each case form a ring together with the atoms joining them,
M² is silicon, germanium or tin,
R⁸ and R⁹ are identical or different and have the meaning mentioned for R¹¹ and
m and n are identical or different and are zero, 1 or 2, m plus n being zero, 1 or 2, and the metallocene of the formula I is different from dimethylsilylenebis (2,4-dimethylindenyl)zirconium dichloride and ethylenebis(2-methyl-4-methylindenyl)hafnium dichloride.

2. A metallocene as claimed in claim 1, in which, in formula I, M¹ is Zr, R¹ and R² are identical or different and are methyl or chlorine, R³ and R⁴ are identical or different and are methyl, ethyl, n-propyl, i-propyl, n-butyl, i-butyl, tert-butyl or neopentyl, R⁵ and R⁶ are identical or different and are methyl or ethyl, R⁷ is a radical 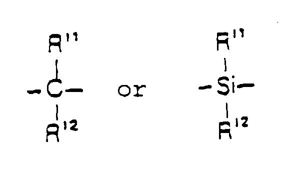 and n plus m is zero or 1.

3. A metallocene as claimed in one or both of claims 1 and 2, wherein, in formula I, the substituents R¹ and R², R³ and R⁴, and R⁵ and R⁶ are in each case identical.

4. rac-Dimethylsilylenebis(1-(2-methyl-4-ethyl-indenyl))zirconiumdichloride, rac-dimethylsilylenebis(1-(2-methyl-4-isopropylindenyl))zirconium dichloride, rac-dimethylsilylenebis(1-(2-methyl-4-tert-butylindenyl))zirconiumdichloride, rac-methyl-phenylsilylenebis(1-(2-methyl-4-isopropylindenyl))zirconium dichloride, rac-dimethylsilylenebis(1-(2-ethyl-4-methylindenyl))zirconium dichloride, rac-dimethylsilylenebis(1-(2-methyl-4-ethylindenyl))zirconium dimethyl.

5. A catalyst which is formed from a metallocene of the formula I, as claimed in one or more of claims 1 to 4, and a cocatalyst.

6. A catalyst as claimed in claim 5, in which the cocatalyst is an aluminoxane of the formula (II) 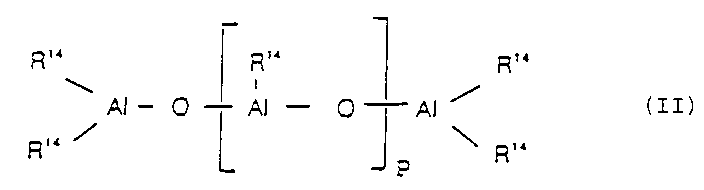 for the linear type and/or of the formula (III) 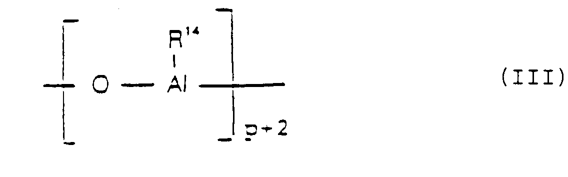 for the cyclic type,
in which, in the formulae (II) and (III), the radicals R¹⁴ are identical or different and are a C₁-C₆-alkyl group, a C₆-C₁₈-aryl group or hydrogen and p is an integer from 2 to 50.

7. A catalyst as claimed in claim 5 or 6, in which the cocatalyst is methylaluminoxane.

8. A catalyst as claimed in one or more of claims 5 to 7, in which the metallocene is applied to a support.

9. A catalyst as claimed in one or more of claims 5 to 8, in which the metallocene is prepolymerized.

10. A process for the preparation of an olefin polymer by polymerization or copolymerization of an olefin of the formula R^{a}-CH=CH-R^{b}, in which R^{a} and R^{b} are identical or different and are a hydrogen atom or a hydrocarbon radical having 1 to 14 carbon atoms, or R^{a} and R^{b} form a ring with the atoms joining them, at a temperature of -60 to 200°C, under a pressure of 0.5 to 100 bar, in solution, in suspension or in the gas phase, in the presence of a catalyst as claimed in one or more of claims 5 to 9.

11. The process as claimed in claim 10, in which ethylene or propylene is polymerized.

12. The process as claimed in claim 10 or 11, in which the metallocene of the formula I is preactivated with an aluminoxane of the formula II and/or III before use in the polymerization reaction.

## Claims (Claims for the following Contracting State(s): ES)

1. A process for the preparation of a metallocene of the formula I 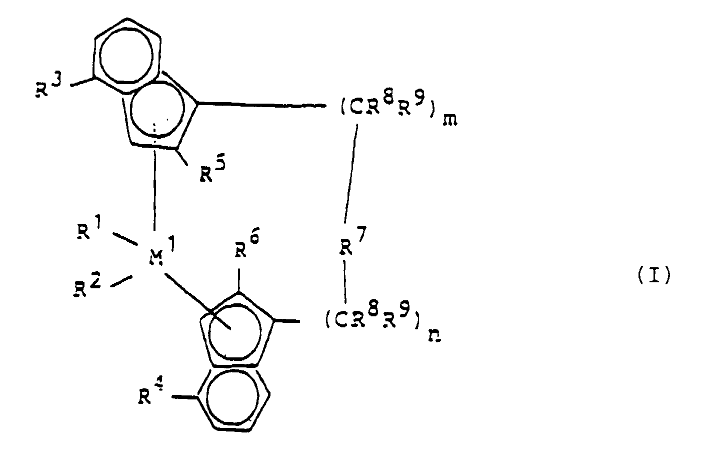 in which
M¹ is a metal of group IVb, Vb or VIb of the Periodic Table,
R¹ and R² are identical or different and are hydrogen, a C₁-C₁₀-alkyl group, a C₁-C₁₀-alkoxy group, a C₆-C₁₀-aryl group, a C₆-C₁₀-aryloxy group, a C₂-C₁₀-alkenyl group, a C₇-C₄₀-arylalkyl group, a C₇-C₄₀-alkylaryl group, a C₈-C₄₀-arylalkenyl group or a haloaen atom.
R³ and R⁴ are identical or different and are a halogen atom, a C₁-C₁₀-alkyl group which may be halogenated, a C₆-C₁₀-aryl group or an -NR₂¹⁰, -SR¹⁰, -OSiR₃¹⁰, -SiR₃¹⁰ or -PR₂¹⁰ radical in which R¹⁰ is a halogen atom, a C₁-C₁₀-alkyl group or a C₆-C₁₀-aryl group,
R⁵ and R⁶ are identical or different and are a halogen atom, a C₁-C₁₀-alkyl group which may be halogenated, a C₆-C₁₀-aryl group or an -NR₂¹⁰, -SR¹⁰, -OSiR₃¹⁰, -SiR₃¹⁰ or -PR₂¹⁰ radical in which R¹⁰ is a halogen atom, a C₁-C₁₀-alkyl group or a C₆-C₁₀-aryl group,
R⁷ is 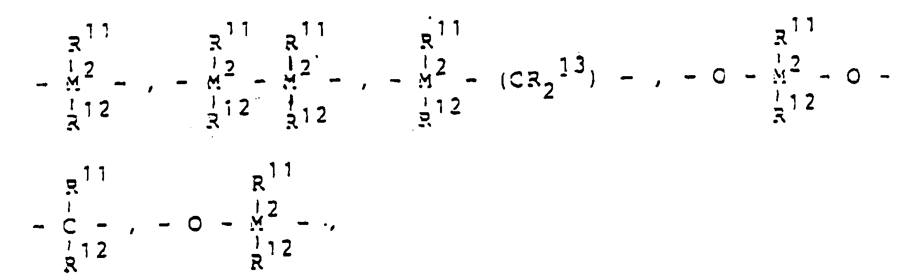 =BR¹¹, =AlR¹¹, -Ge-, -Sn-, -O-, -S-, =SO, =SO₂, =NR¹¹, =CO, =PR¹¹ or =P(O)R¹¹,
in which
R¹¹, R¹² and R¹³ are identical or different and are a hydrogen atom, a halogen atom, a C₁-C₁₀-alkyl group, a C₁-C₁₀-fluoroalkyl group, a C₆-C₁₀-aryl group, a C₆-C₁₀-fluoroaryl group, a C₁-C₁₀-alkoxy group, a C₂-C₁₀-alkenyl group, a C₇-C₄₀-arylalkyl group, a C₈-C₄₀-arylalkenyl group or a C₇-C₄₀-alkylaryl group, or R¹¹ and R¹² or R¹¹ and R¹³ in each case form a rina with the atoms joining them, or R¹¹ or R¹² with R⁸ or R⁹ in each case form a ring together with the atoms joining them,
M² is silicon, germanium or tin,
R⁸ and R⁹ are identical or different and have the meaning mentioned for R¹¹ and
m and n are identical or different and are zero, 1 or 2, m plus n being zero, 1 or 2, and the metallocene of the formula I is different from dimethylsilylenebis(2,4-dimethylindenyl)-zirconium dichloride and ethylenebis(2-methyl-4-methylindenyl)hafnium dichloride.

2. The process as claimed in claim 1, in which, in formula I, M¹ is Zr, R¹ and R² are identical or different and are methyl or chlorine, R³ and R⁴ are identical or different and are methyl, ethyl, n-propyl, i-propyl, n-butyl, i-butyl, tert-butyl or neopentyl, R⁵ and R⁶ are identical or different and are methyl or ethyl, R⁷ is a radical 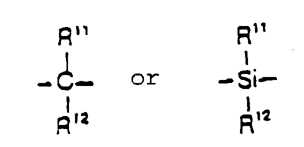 and n plus m is zero or 1.

3. The process as claimed in one or both of claims 1 and 2, wherein, in formula I, the substituents R¹ and R², R³ and R⁴, and R⁵ and R⁶ are in each case identical.

4. The process as claimed in one or more of claims 1 to 3, in which the metallocene is chosen from rac-dimethylsilylenebis(1-(2-methyl-4-ethylindenyl))zirconium dichloride, rac-dimethylsilylenebis (1-(2-methyl-4-isopropylindenyl))zirconium dichloride. rac-dimethylsilylenebis(1-(2-methyl-4-tert-butylindenyl))zirconium dichloride, rac-methylphenylsilylenebis(1-(2-methyl-4-isopropylindenyl))zirconium dichloride, rac-dimethylsilylenebis(1-(2-ethyl-4-methylindenyl))zirconium dichloride, rac-dimethylsilylenebis(1-(2-methyl-4-ethylindenyl))zirconium dimethyl.

5. A process for the preparation of a catalyst which is formed from a metallocene of the formula I, as claimed in one or more of claims 1 to 4, and a cocatalyst.

6. The process as claimed in claim 5, in which the cocatalyst is an aluminoxane of the formula (II) 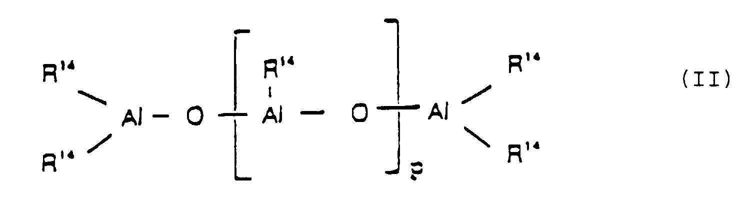 for the linear type and/or of the formula (III) 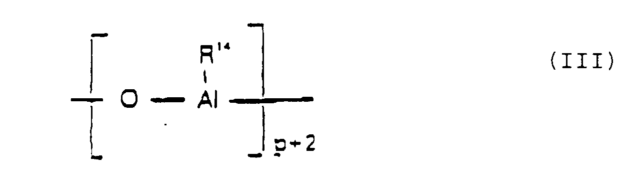 for the cyclic type,
in which, in the formulae (II) and (III), the radicals R¹⁴ are identical or different and are a C₁-C₆-alkyl group, a C₆-C₁₈-aryl group or hydrogen and p is an integer from 2 to 50.

7. The process as claimed in claim 5 or 6, in which the cocatalyst is methylaluminoxane.

8. The process as claimed in one or more of claims 5 to 7, in which the metallocene is applied to a support.

9. The process as claimed in one or more of claims 5 to 8, in which the metallocene is prepolymerized.

10. A process for the preparation of an olefin polymer by polymerization or copolymerization of an olefin of the formula R^{a}-CH=CH-R^{b}, in which R^{a} and R^{b} are identical or different and are a hydrogen atom or a hydrocarbon radical having 1 to 14 carbon atoms, or R^{a} and R^{b} form a ring with the atoms joining them, at a temperature of -60 to 200°C, under a pressure of 0.5 to 100 bar, in solution, in suspension or in the gas phase, in the presence of a catalyst, as claimed in one or more of claims 5 to 9.

11. The process as claimed in claim 10, in which ethylene or propylene is polymerized.

12. The process as claimed in claim 10 or 11, in which the metallocene of the formula I is preactivated with an aluminoxane of the formula II and/or III before use in the polymerization reaction.

## Revendications (Revendications pour l'(les) Etat(s) contractant(s) suivant(s): AT, BE, CH, LI, DE, FR, GB, IT, LU, NL, SE)

1. Métallocène de formule I : 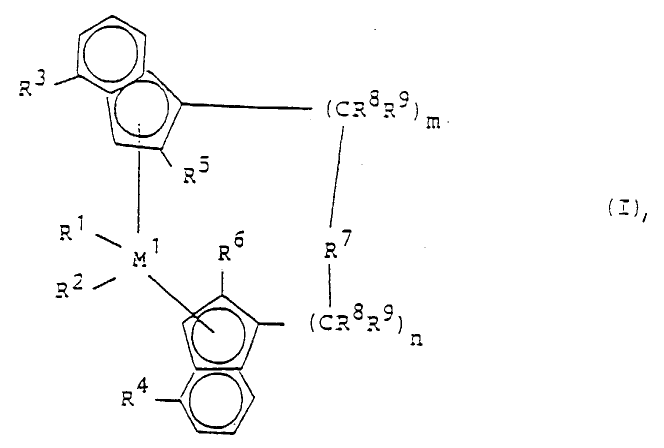 dans laquelle :
M¹ est un métal du groupe IVb, Vb ou VIb du Tableau Périodique,
R¹ et R² sont identiques ou différents et représentent chacun un hydrogène ou un groupe alkyle en C₁-C₁₀, un groupe alcoxy en C₁-C₁₀, un groupe aryle en C₆-C₁₀, un groupe aryloxy en C₆-C₁₀, un groupe alcényle en C₂-C₁₀, un groupe arylalkyle en C₇-C₄₀, un groupe arylalkyle en C₇-C₄₀, un groupe arylalcényle en C₈-C₄₀ ou un atome d'halo-gène,
R³ et R⁴ sont identiques ou différents et représentent chacun un atome d'halogène, un groupe alkyle en C₁-C₁₀, qui peut être halogéné, un groupe aryle en C₈-C₁₀, un radical -NR₂¹⁰, -SR¹⁰, -OSiR₃¹⁰, SiR₃¹⁰ ou -PR₂¹⁰, où R¹⁰ est un atome d'halogène, un groupe alkyle en C₁-C₁₀ ou un groupe aryle en C₆-C₁₀,
R⁵ et R⁶ sont identiques ou différents et représentent chacun un atome d'halogène, un groupe alkyle en C₁-C₁₀, qui peut être halogéné, un groupe aryle en C₈-C₁₀, un radical -NR₂¹⁰, -SR¹⁰, -OSiR₃¹⁰, SiR₃¹⁰ ou -PR₂¹⁰, où R¹⁰ est un atome d'halogène, un groupe alkyle en C₁-C₁₀, ou un groupe aryle en C₆-C₁₀,
R⁷ est : 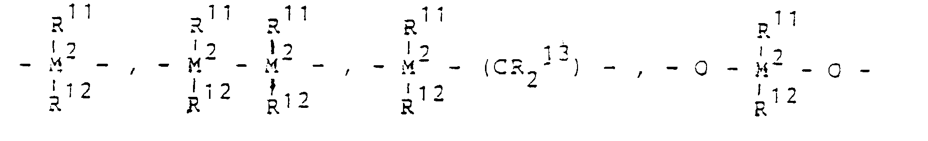 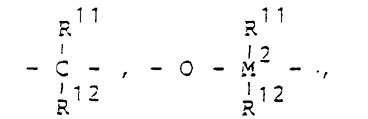
=BR¹¹, =AlR¹¹, -Ge-, -Sn-, -O-, -S-, =SO₂ =NR¹¹, =CO, =PR¹¹ ou =P(O)R¹¹,
où :
R¹¹, R¹² et R¹³ sont identiques ou différents et représentent chacun un atome d'hydrogène, un atome d'halogène, un groupe alkyle en C₁-C₁₀, un groupe fluoralkyle en C₁-C₁₀, un groupe aryle en C₆-C₁₀, un groupe fluoraryle en C₆-C₁₀, un groupe alcoxy en C₁-C₁₀, un groupe alcényle en C₂-C₁₀, un groupe arylalkyle en C₇-C₄₀, un groupe arylalcényle en C₈-C₄₀, un groupe arylalkyle en C₇-C₄₀, ou encore R¹¹ et R¹² ou R¹¹ et R¹³, chacun avec les atomes qui les relient, forment un noyau, ou bien encore R¹¹ et R¹², avec R⁸ et R⁹, forment chacun avec les atomes qui relient un noyau,
M² est le silicium, le germanium ou l'étain,
R⁸ et R⁹ sont identiques ou différents et ont les significations données pour R¹¹, et
m et n sont identiques ou différents et valent chacun 0, 1 ou 2, m+n valant 0, 1 ou 2, et le métallocène de formule 1 est différent du dichlorure de diméthylsilylènebis (2,4-diméthylindényl)zirconium et du dichlorure d'éthylènebis (2-méthyl-4-méthylindényl)-hafnium,

2. Métallocène selon la revendication 1, dans lequel, dans la formule I, M¹ est Zr, R¹ et R² sont identiques ou différents et sont chacun le groupe méthyle ou chloro, R³ et R⁴ sont identiques ou différents et représentent chacun le groupe méthyle, éthyle, n-propyle, isopropyle, n-butyle, isobutyle, tert-butyle ou néo-pentyle, R⁵ et R⁶ sont identiques ou différents et représentent chacun le groupe méthyle ou éthyle, R⁷ est un radical : 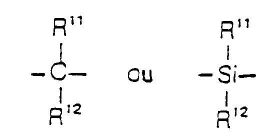 et la somme n+m vaut de 0 ou 1.

3. Métallocène selon une ou plusieurs des revendications 1 ou 2, caractérisé en ce que, dans la formule I, les substituants R¹ et R², R³ et R⁴, et R⁵ et R⁶, sont identiques deux à deux.

4. Dichlorure de rac-diméthylsilylène-bis(1-(2-méthyl-4-éthylindényl)zirconium, dichlorure de rac-diméthylsilylène-bis(1-(2-méthyl-4-isopropylindényl)zirconium, dichlorure de rac-diméthylsilylène-bis(1-(2-méthyl-4-tert-butylindényl)-zirconium, dichlorure de rac-méthylphénylsilylène-bis(1-(2-méthyl-4-isopropylindényl)zirconium, dichlorure de rac-diméthylsilylènebis(1-(2-éthyl-4-méthylindényl)-zirconium, dichlorure de diméthyl-rac-diméthylsilylène-bis(1-(2-méthyl-4-éthylindényl)zirconium.

5. Catalyseur qui est constitué d'un métallocène de formule I selon l'une ou plusieurs des revendications 1 à 4, et d'un co-catalyseur.

6. Catalyseur selon la revendication 5, dans lequel le co-catalyseur est un aluminoxane de formule II : 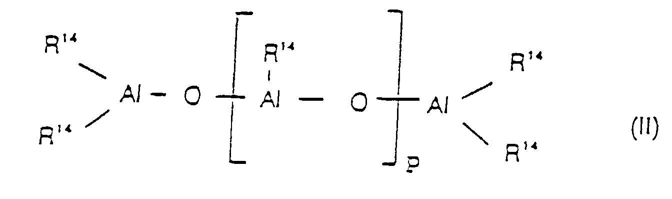 pour le type linéaire, et/ou de formule III : 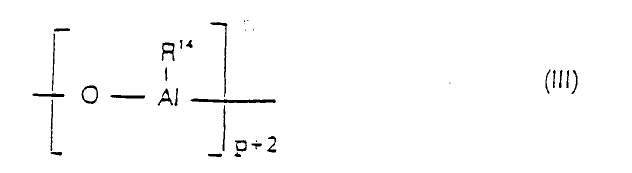 pour le type cyclique,
où :
dans les formules (II) et (III), les radicaux R¹⁴ sont identiques ou différents et représentent chacun un groupe alkyle en C₁-C₆, un groupe aryle en C₆-C₁₈, ou un atome d'hydrogène, et p est un nombre entier de 2 à 50.

7. Catalyseur selon la revendication 5 ou 6, dans lequel le co-catalyseur est le méthylaluminoxane.

8. Catalyseur selon l'une ou plusieurs des revendications 5 à 7, dans lequel le métallocène est appliqué sur un support.

9. Catalyseur selon l'une ou plusieurs des revendications 5 à 8, dans lequel le métallocène est prépolymérisé.

10. Procédé de préparation d'un polymère oléfinique par polymérisation ou copolymérisation d'une oléfine de formule R^{a}-CH=CH-R^{b}, où R^{a} et R^{b} sont identiques ou différents et représentent chacun un atome d'hydrogène ou un radical hydrocarboné ayant de 1 à 14 atomes de carbone, ou bien R^{a} et R^{b} forment un cycle avec les atomes qui les relient, à une température de -60 à 200°C, sous une pression de 0,5 à 100 bar, en solution, en suspension ou en phase gazeuse, en présence d'un catalyseur selon l'une ou plusieurs des revendications 5 à 9.

11. Procédé selon la revendication 10, dans lequel la polymérisation porte sur l'éthylène ou le propylène.

12. Procédé selon la revendication 10 ou 11, dans lequel le métallocène de formule I est, avant utilisation dans la réaction de polymérisation, préactivé avec un aluminoxane de formule II et/ou III.

## Revendications (Revendications pour l'(les) Etat(s) contractant(s) suivant(s): ES)

1. Procédé de préparation de métallocène de formule I : 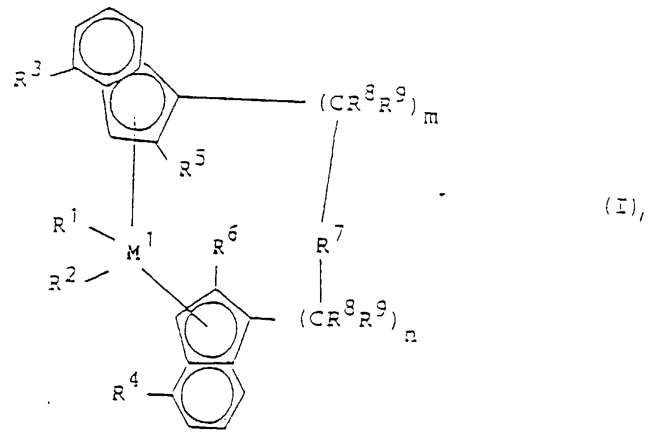 dans laquelle :
M¹ est un métal du groupe IVb, Vb ou VIb du Tableau Périodique,
R¹ et R² sont identiques ou différents et représentent chacun un hydrogène ou un groupe alkyle en C₁-C₁₀, un groupe alcoxy en C₁-C₁₀, un groupe aryle en C₆-C₁₀, un groupe aryloxy en C₆-C₁₀, un groupe alcényle en C₂-C₁₀, un groupe arylalkyle en C₇-C₄₀, un groupe arylalkyle en C₇-C₄₀, un groupe arylalcényle en C₈-C₄₀ ou un atome d'halogène,
R³ et R⁴ sont identiques ou différents et représentent chacun un atome d'halogène, un groupe alkyle en C₁-C₁₀, qui peut être halogéné, un groupe aryle en C₈-C₁₀, un radical -NR₂¹⁰, -SR¹⁰, -OSiR₃¹⁰, SiR₃¹⁰ ou -PR₂¹⁰, où R¹⁰ est un atome d'halogène, un groupe alkyle en C₁-C₁₀ ou un groupe aryle en C₆-C₁₀,
R⁵ et R⁶ sont identiques ou différents et représentent chacun un atome d'halogène, un groupe alkyle en C₁-C₁₀, qui peut être halogéné, un groupe aryle en C₈-C₁₀, un radical -NR₂¹⁰, -SR¹⁰, -OSiR₃¹⁰, SiR₃¹⁰ ou -PR₂¹⁰, où R¹⁰ est un atome d'halogène, un groupe alkyle en C₁-C₁₀, ou un groupe aryle en C₆-C₁₀,
R¹⁰ est : 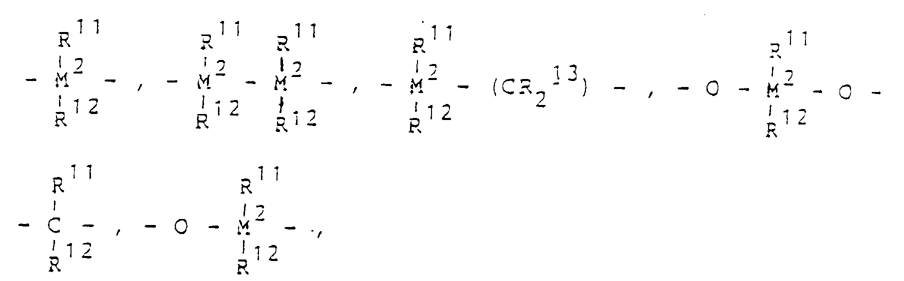
=BR¹¹, =AlR¹¹, -Ge-, -Sn-, -O-, -S-, =SO₂ =NR¹¹, =CO, =PR¹¹ ou =P(O)R¹¹,
où :
R¹¹, R¹² et R¹³ sont identiques ou différents et représentent chacun un atome d'hydrogène, un atome d'halogène, un groupe alkyle en C₁-C₁₀, un groupe fluoralkyle en C₁-C₁₀, un groupe aryle en C₆-C₁₀, un groupe fluoraryle en C₆-C₁₀, un groupe alcoxy en C₁-C₁₀, un groupe alcényle en C₂-C₁₀, un groupe arylalkyle en C₇-C₄₀, un groupe arylalcényle en C₈-C₄₀, un groupe arylalkyle en C₇-C₄₀, ou encore R¹¹ et R¹² ou R¹¹ et R¹³, chacun avec les atomes qui les relient, forment un noyau, ou bien encore R¹¹ ou R¹², avec R⁸ ou R⁹, forment chacun avec les atomes qui relient un noyau,
M² est le silicium, le germanium ou l'étain,
R⁸ et R⁹ sont identiques ou différents et ont les significations données pour R¹¹, et
m et n sont identiques ou différents et valent chacun 0, 1 ou 2, m+n valant 0, 1 ou 2, et le métallocène de formule 1 est différent du dichlorure de diméthylsilylènebis (2,4-diméthylindényl)zirconium et du dichlorure d'éthylènebis(2-méthyl-4-méthylindényl)-hafnium,

2. Métallocène selon la revendication 1, dans lequel, dans la formule I, M¹ est Zr, R¹ et R² sont identiques ou différents et sont chacun le groupe méthyle ou chloro, R³ et R⁴ sont identiques ou différents et représentent chacun le groupe méthyle, éthyle, n-propyle, isopropyle, n-butyle, isobutyle, tert-butyle ou néo-pentyle, R⁵ et R⁶ sont identiques ou différents et représentent chacun le groupe méthyle ou éthyle, R⁷ est un radical : 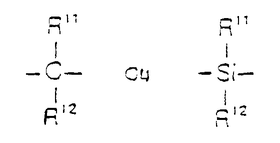 et la somme n+m vaut de 0 ou 1.

3. Procédé selon une ou plusieurs des revendications 1 ou 2, caractérisé en ce que, dans la formule I, les substituants R¹ et R², R³ et R⁴, et R⁵ et R⁶, sont identiques deux à deux.

4. Procédé selon une ou plusieurs des revendication 1 à 3, où on prend le métallocène dans le groupe comportant les : dichlorure de rac-diméthylsilylène-bis(1-(2-méthyl-4-éthylindényl)zirconium, dichlorure de rac-diméthylsilylènebis(1-(2-méthyl-4-isopropylindényl)zirconium, dichlorure de rac-diméthylsilylène-bis(1-(2-méthyl-4-tert-butylindényl)zirconium, dichlorure de rac-méthylphénylsilylène-bis(1-(2-méthyl-4-isopropylindényl)zirconium, dichlorure de rac-diméthylsilylènebis(1-(2-éthyl-4-méthylindényl)-zirconium, dichlorure de diméthyl-rac-diméthylsilylènebis(1-(2-méthyl-4-éthylindényl)zirconium.

5. Procédé pour la préparation d'un catalyseur qui est constitué d'un métallocène de formule I selon l'une ou plusieurs des revendications 1 à 4, et d'un co-catalyseur.

6. Procédé selon la revendication 5, dans lequel le co-catalyseur est un aluminoxane de formule II : 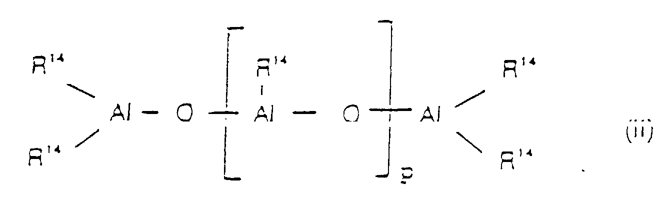 pour le type linéaire, et/ou de formule III : 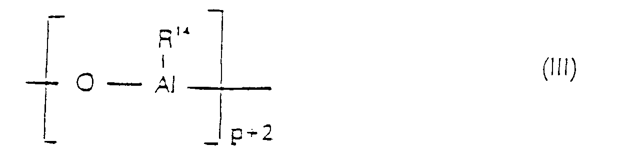 pour le type cyclique,
où :
dans les formules (II) et (III), les radicaux R¹⁴ sont identiques ou différents et représentent chacun un groupe alkyle en C₁-C₆, un groupe aryle en C₆-C₁₈, ou un atome d'hydrogène, et p est un nombre entier de 2 à 50.

7. Procédé selon la revendication 5 ou 6, dans lequel le co-catalyseur est le méthylaluminoxane.

8. Procédé selon l'une ou plusieurs des revendications 5 à 7, dans lequel le métallocène est appliqué sur un support.

9. Procédé selon l'une ou plusieurs des revendications 5 à 8, dans lequel le métallocène est prépolymérisé.

10. Procédé de préparation d'un polymère oléfinique par polymérisation ou copolymérisation d'une oléfine de formule R^{a}-CH=CH-R^{b}, où R^{a} et R^{b} sont identiques ou différents et représentent chacun un atome d'hydrogène ou un radical hydrocarboné ayant de 1 à 14 atomes de carbone, ou bien R^{a} et R^{b} forment un cycle avec les atomes qui les relient, à une température de -60 à 200°C, sous une pression de 0,5 à 100 bar, en solution, en suspension ou en phase gazeuse, en présence d'un catalyseur selon l'une ou plusieurs des revendications 5 à 9.

11. Procédé selon la revendication 10, dans lequel la polymérisation porte sur l'éthylène ou le propylène.

12. Procédé selon la revendication 10 ou 11, dans lequel le métallocène de formule I est, avant utilisation dans la réaction de polymérisation, préactivé avec un aluminoxane de formule II et/ou III.
